# EUROPÄISCHE PATENTANMELDUNG

(11) **EP 0 690 061 A1**
(43) Veröffentlichungstag der Anmeldung: **03.01.1996**
(21) Anmeldenummer: 95110008.0
(22) Anmeldetag: 27.06.1995
(51) Int. Cl.: C07D 513/04, A01N 43/90

(54) **Substituierte Pyridobenzothiadiazole, Verfahren und Zwischenprodukte zu ihrer Herstellung und ihre Verwendung als Mikrobizide**

(30) Priorität: 30.06.1994 CH 2093/94
(71) Anmelder: CIBA-GEIGY AG, CH-4002 Basel (CH)
(72) Erfinder: Maetzke, Thomas, Dr., CH-4142 Münchenstein (CH)
(74) Vertreter: Zumstein, Fritz, Dr.

(57) **Zusammenfassung**

Verbindungen der Formel I 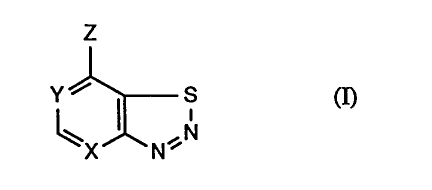
worin
a) X CH und
   Y N; oder
b) X N und
   Y CH; bedeuten und worin
   Z eine C₁-Gruppe darstellt, an die 1-3 Halogenatome oder 1-3 gegebenenfalls substituierte Heteroatome O,S und/oder N gebunden sind,
   eignen sich zur Bekämpfung und Verhütung von Mikroorganismenbefall an Pflanzern.

## Beschreibung

Die Erfindung betrifft neue mikrobizid wirksame Verbindungen der Formel I, 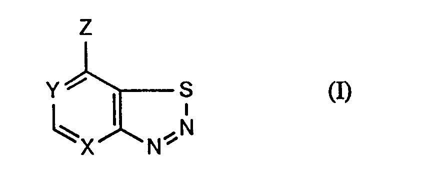
worin
a) X CH und
   Y N; oder
b) X N und
   Y CH; bedeuten und worin
   Z eine C₁-Gruppe darstellt, an die 1-3 Halogenatome oder 1-3 gegebenenfalls substituierte Heteroatome O,S und/oder N gebunden sind;
   in freier Form oder in Salzform.

Die Erfindung betrifft ferner die Herstellung dieser Verbindungen, agrochemische Mittel, die als Wirkstoff mindestens eine dieser Verbindungen enthalten, sowie die Verwendung der Wirkstoffe oder der Mittel zum Schutz von Pflanzen gegen den Befall durch schädliche Mikroorganismen, insbesondere pflanzenschädigende Pilze.

Die Verbindungen der Formel I und gegebenenfalls ihre Tautomeren können als Salze vorliegen. Verbindungen der Formel I, die mindestens ein basisches Zentrum aufweisen, können Säureadditionssalze bilden. Diese werden beispielsweise mit Mineralsäuren, z.B. Schwefelsäure, einer Phosphorsäure oder einer Halogenwasserstoffsäure, mit organischen Carbonsäuren, wie z.B. Essigsäure, Oxal-, Malon-, Malein-, Fumar- oder Phthalsäure, mit Hydroxycarbonsäuren, z.B. Ascorbin-, Milch-, Äpfel-, Wein- oder Zitronensäure, oder mit Benzoesäure, oder mit organischen Sulfonsäuren, wie z.B. Methan- oder p-Toluolsulfonsäure, gebildet.
Ferner können Verbindungen der Formel I mit mindestens einer aciden Gruppe Salze mit Basen bilden. Geeignete Salze mit Basen sind beispielsweise Metallsalze, wie Alkali- oder Erdalkalimetallsalze, z.B. Natrium-, Kalium- oder Magnesiumsalze, oder Salze mit Ammoniak oder einem organischen Amin, wie Morpholin, Piperidin, Pyrrolidin, einem Mono-, Di- oder Triniederalkylamin, z. B. Ethyl-, Diethyl-, Triethyl- oder Dimethyl-propyl-amin, oder einem Mono-, Di- oder Trihydroxyniederalkylamin, z.B. Mono-, Di- oder Triethanolamin. Weiterhin können gegebenenfalls entsprechende innere Salze gebildet werden. Bevorzugt sind im Rahmen der Erfindung agrochemisch vorteilhafte Salze.

Die vor- und nachstehend verwendeten Allgemeinbegriffe haben, sofern nicht abweichend definiert, die nachfolgend aufgeführten Bedeutungen:
Kohlenwasserstoffreste können gesättigt oder ungesättigt, offenkettig oder cyclisch, oder gemischt offenkettig und cyclisch, wie z.B. Cyclopropylmethyl oder Benzyl, sein.

Alkylgruppen sind, je nach Anzahl der Kohlenstoffatome, geradkettig oder verzweigt und stehen beispielsweise für Methyl, Ethyl, n-Propyl, iso-Propyl, n-Butyl, sek.-Butyl, iso-Butyl, tert.-Butyl, sek. Amyl, tert. Amyl, 1-Hexyl oder 3-Hexyl.

Ungesättigte Kohlenwasserstoffreste sind Alkenyl-, Alkinyl- oder Alkeninylgruppen mit höchstens 3 Mehrfachbindungen, wie z.B. Butadienyl, Hexatrienyl, 2-Penten-4-inyl.

Unter Alkenyl ist geradkettiges oder verzweigtes Alkenyl zu verstehen, wie z.B. Allyl, Methallyl, 1-Methylvinyl oder But-2-en-1-yl. Bevorzugt sind Alkenylreste mit einer Kettenlänge von 3 bis 4 Kohlenstoffatomen.

Alkinyl kann ebenfalls, je nach Anzahl der Kohlenstoffatome, geradkettig oder verzweigt sein, wie z.B. Propargyl, But-1-in-1-yl, But-1-in-3-yl. Bevorzugt ist Propargyl.

Cyclische ungesättigte Kohlenwasserstoffreste können aromatisch sein wie z.B. Phenyl und Naphtyl, oder nicht aromatisch wie z.B. Cyclopentenyl, Cyclohexenyl, Cycloheptenyl und Cyclooctadienyl, oder teilaromatisch wie z.B. Tetrahydronaphtyl und Indanyl.

Halogen bzw. Halo stehen für Fluor, Chlor, Brom oder Jod, vorzugsweise für Fluor, Chlor oder Brom.

Haloalkyl kann gleiche oder verschiedene Halogenatome enthalten, z.B. Fluormethyl, Difluormethyl, Difluorchlormethyl, Trifluormethyl, Chlormethyl, Dichlormethyl, Trichlormethyl, 2,2,2-Trifluorethyl, 2-Fluorethyl, 2-Chlorethyl, 2,2,2-Trichlorethyl, 3,3,3-Trifluorpropyl.

Alkoxy ist beispielsweise Methoxy, Ethoxy, Propyloxy, i-Propyloxy, n-Butyloxy, iso-Butyloxy, sek.-Butyloxy und tert.-Butyloxy; vorzugsweise Methoxy und Ethoxy.

Haloalkoxy ist z.B. Difluormethoxy, Trifluormethoxy, 2,2,2-Trifluorethoxy, 1,1,2,2-Tetrafluorethoxy, 2-Fluorethoxy, 2-Chlorethoxy und 2,2-Difluorethoxy.

Cycloalkyl ist je nach Ringgrösse Cyclopropyl, Cyclobutyl, Cyclopentyl oder Cyclohexyl.

Alkanoyl ist, jeweils unter Berücksichtigung der von Fall zu Fall umfassten Anzahl Kohlenstoffatome, entweder geradkettig oder verzweigt, Beispiele sind Formyl, Acetyl, Propionyl, Butyryl, Pivaloyl oder Octanoyl.

Unter einem Heterocyclylrest werden 5- oder 6-gliedrige, aromatische und nicht aromatische Ringe mit Heteroatomen N, O und/oder S verstanden. Weiterhin kann einem solchen an das Restmolekül gebundenen Heterocyclylrest eine unsubstituierte oder substituierte Benzo-Gruppe ankondensiert sein. Beispiele für Heterocyclylgruppen sind Pyridyl, Pyrimidinyl, Imidazolyl, Thiazolyl, 1,3,4-Thiadiazolyl, Triazolyl, Thienyl, Furanyl, Pyrrolyl, Morpholinyl, Oxazolyl und die entsprechenden partiell oder total hydrierten Ringe. Beispiele für Heterocyclylgruppen mit ankondensierter Benzogruppe sind Chinolyl, Isochinolyl, Benzoxazolyl, Chinoxalinyl, Benzothiazolyl, Benzimidazolyl, Indolyl, Indolinyl.

Alle vorgenannten Aufzählungen sind beispielhaft und stellen keine Limitierungen dar.

Bevorzugte Gruppen sind:
(1) Verbindungen der Formel I, worin bedeuten:
   Z CH, CO-A, CS-A, CH=U, CH₂-V, CH(-V)₂ oder C(-V)₃;
   und worin ferner die übrigen Substituenten folgende Bedeutungen haben:
   A OR₂, SR₂, N(R₃)R₄, N(R₅)-OR₆, O-N(=C)ₙ(R₇)R₈ oder N(R₉)-N(=C)ₙ(R₇)R₈;
   U O, NR₁₀, N-OR₆ oder N-N(=C)ₙ(R₇)R₈;
   V Halogen, Hydroxy, C₁-C₄-Alkoxy, C₁-C₄-Acyloxy, Benzoyloxy, Benzyloxy oder zwei V zusammen mit dem Kohlenstoffatom, an das sie gebunden sind, eine cyclische Acetalgruppe;
   n 0 oder 1;
   R₂ bis R₁₀ Wasserstoff, ein gegebenenfalls substituierter, bis zu 8 Kohlenstoffatome enthaltender offenkettiger, gesättigter oder ungesättigter Kohlenwasserstoffrest, ein gegebenenfalls substituierter, bis zu 10 Kohlenstoffatome enthaltender cyclischer, gesättigter oder ungesättigter Kohlenwasserstoffrest, gegebenenfalls substituiertes Benzyl oder Phenethyl, eine gegebenenfalls substituierte, bis zu 8 Kohlenstoffatome enthaltende Acylgruppe, eine gegebenenfalls substituierte Benzoylgruppe oder ein gegebenenfalls substituierter Heterocyclylrest; oder
   R₃ und R₄ zusammen mit dem Stickstoffatom, an das sie gebunden sind, einen 5- oder 6-gliedrigen, gegebenenfalls substituierten Heterocyclus; oder
   R₇ und R₈ zusammen mit dem Atom, an welches sie gebunden sind, einen 5- oder 6-gliedrigen carbocyclischen oder heterocyclischen Ring, wobei die genannten Ringe unsubstituiert oder substituiert sind;
   in freier Form oder in Salzform.
(2) Verbindungen der Formel I, worin bedeuten:
   R₂ und R₃ Wasserstoff, C₁-C₈-Alkyl, das unsubstituiert oder mit 1-5 Halogenatomen, C₃-C₆-Cycloalkyl, C₁-C₄-Alkoxy, Phenoxy, Hydroxy, Nitro, Cyano, C₁-C₄-Alkanoyl, Benzoyl, Carboxyl, C₁-C₄-Alkoxycarbonyl, Benzyloxycarbonyl, C₁-C₄-Acyloxy, Benzoyloxy, C₁-C₄-Dialkylamino oder Heterocyclyl substituiert ist, C₃-C₆-Alkenyl, das unsubstituiert oder mit 1-5 Halogenatomen substituiert ist, C₃-C₆-Alkinyl, C₃-C₆-Cycloalkyl, Phenyl, Benzyl oder Phenethyl, deren Phenylringe unsubstitiert oder ein bis dreifach durch Halogen, Hydroxy, C₁-C₄-Alkyl, Halogen-C₁-C₂-alkyl, C₁-C₂-Alkoxy, Halogen-C₁-C₂-alkoxy oder Nitro substituiert sind, Naphthyl, C₁-C₄-Alkanoyl, Benzoyl, oder Heterocyclyl, das unsubstituiert oder ein- bis dreifach gleich oder verschieden durch Halogen, C₁-C₂-Alkyl, Halogenmethyl oder Nitro substituiert ist;
   R₄ Wasserstoff, C₁-C₆-Alkyl, C₃-C₆-Alkenyl, C₃-C₆-Alkinyl, C₃-C₆-Cycloalkyl, Phenyl oder Benzyl, deren Phenylringe unsubstitiert oder ein bis zweifach durch Halogen, Hydroxy, C₁-C₂-Alkyl, C₁-C₂-Alkoxy, Halogenmethyl oder Nitro substituiert sind; oder
   R₃ und R₄ zusammen mit dem Stickstoffatom, an das sie gebunden sind, einen 5- oder 6-gliedrigen Heterocyclus, der unsubstituiert oder ein- bis dreifach durch C₁-C₂-Alkyl substituiert ist;
   R₅, R₆, R₇, R₈, R₉ und R₁₀ unabhängig voneinander Wasserstoff, C₁-C₆-Alkyl, C₃-C₆-Alkenyl, C₃-C₆-Alkinyl, C₃-C₆-Cycloalkyl, Phenyl oder Benzyl, deren Phenylringe unsubstitiert oder ein bis zweifach durch Halogen, Hydroxy, C₁-C₂-Alkyl, C₁-C₂-Alkoxy, Halogen-C₁-C₂-alkyl oder Nitro substituiert sind;
   oder
   R₇ und R₈ zusammen mit dem Atom, an welches sie gebunden sind, einen 5- bis 7-gliedrigen carbocyclischen oder heterocyclischen Ring, wobei die genannten Ringe unsubstituiert oder ein- bis dreifach gleich oder verschieden durch Halogen, C₁-C₂-Alkyl, Halogen-C₁-C₂-alkyl oder Nitro substituiert sind.
(3) Verbindungen der Formel I, worin bedeuten:
   R₂ und R₃ Wasserstoff, C₁-C₅-Alkyl, das unsubstituiert oder mit 1-3 Halogenatomen, C₃-C₆-Cycloalkyl, C₁-C₂-Alkoxy, Phenoxy, Hydroxy, Nitro, Cyano, C₁-C₂-Alkanoyl, Benzoyl, Carboxyl, C₁-C₄-Alkoxycarbonyl, Benzyloxycarbonyl, C₁-C₂-Acyloxy, Benzoyloxy, C₁-C₄-Dialkylamino oder Heterocyclyl substituiert ist, C₃-C₄-Alkenyl, das unsubstituiert oder mit 1-3 Halogenatomen substituiert ist, C₃-C₄-Alkinyl, C₃-C₆-Cycloalkyl, Phenyl, Benzyl oder Phenethyl, deren Phenylringe unsubstitiert oder ein bis zweifach durch Halogen, Hydroxy, C₁-C₄-Alkyl, Halogen-C₁-C₂-alkyl, C₁-C₂-Alkoxy, Halogen-C₁-C₂-alkoxy, oder Nitro substituiert sind, Naphthyl oder Heterocyclyl, das unsubstituiert oder ein- bis zweifach gleich oder verschieden durch Halogen, C₁-C₂-Alkyl, Halogenmethyl oder Nitro substituiert ist;
   R₄ Wasserstoff, C₁-C₄-Alkyl, C₃-C₄-Alkenyl, C₃-C₄-Alkinyl, C₃-C₆-Cycloalkyl, Phenyl oder Benzyl;
   R₃ und R₄ zusammen mit dem Stickstoffatom, an das sie gebunden sind, Pyrrolidin, Piperidin, Morpholin oder Dimethylmorpholin;
   R₅, R₆, R₇, R₈, R₉ und R₁₀ unabhängig voneinander Wasserstoff, C₁-C₄-Alkyl, C₃-C₄-Alkenyl, C₃-C₄-Alkinyl, C₃-C₆-Cycloalkyl, Phenyl oder Benzyl, deren Phenylringe unsubstitiert oder ein bis zweifach durch Halogen, Hydroxy, C₁-C₂-Alkyl, C₁-C₂-Alkoxy, Halogenmethyl oder Nitro substituiert sind; oder
   R₇ und R₈ zusammen mit dem Atom, an welches sie gebunden sind, einen 5- bis 7-gliedrigen carbocyclischen oder heterocyclischen Ring, wobei die genannten Ringe unsubstituiert oder ein- bis zweifach gleich oder verschieden durch Halogen, Methyl, Halogenmethyl oder Nitro substituiert sind.
(4) Verbindungen der Formel I, worin bedeuten:
   Z CO-A;
   A OR₂, SR₂, N(R₃)R₄, N(R₅)-OR₆, O-N(=C)ₙ(R₇)R₈ oder N(R₉)-N(=C)ₙ(R₇)R₈; davon besonders solche, worin
   A OR₂ oder SR₂ bedeutet;
   worin R₂ bis R₉ die in Formel I angegebenen Bedeutungen haben.
(5) Von den unter (4) genannten Verbindungen sind diejenigen bevorzugt, worin
   R₂ Wasserstoff, C₁-C₅-Alkyl, das unsubstituiert oder mit 1-3 Halogenatomen, C₃-C₆-Cycloalkyl, C₁-C₂-Alkoxy, Phenoxy, Hydroxy, Nitro, Cyano, C₁-C₂-Alkanoyl, Benzoyl, Carboxyl, C₁-C₄-Alkoxycarbonyl, Benzyloxycarbonyl, C₁-C₂-Acyloxy, Benzoyloxy, C₁-C₄-Dialkylamino oder Heterocyclyl substituiert ist, C₃-C₄-Alkenyl, das unsubstituiert oder mit 1-3 Halogenatomen substituiert ist, C₃-C₄-Alkinyl, C₃-C₆-Cycloalkyl, Phenyl, Benzyl oder Phenethyl, deren Phenylringe unsubstitiert oder ein bis zweifach durch Halogen, Hydroxy, C₁-C₄-Alkyl, Halogen-C₁-C₂-alkyl, C₁-C₂-Alkoxy, Halogen-C₁-C₂-alkoxy, oder Nitro substituiert sind, Naphthyl oder Heterocyclyl, das unsubstituiert oder ein- bis zweifach gleich oder verschieden durch Halogen, C₁-C₂-Alkyl, Halogenmethyl oder Nitro substituiert ist;
   besonders bevorzugt diejenigen, worin bedeuten:
   R₂ Wasserstoff, C₁-C₅-Alkyl,Propenyl, Phenyl oder Benzyl, deren Phenylringe unsubstitiert oder ein bis zweifach durch Halogen substituiert sind.
(6) Verbindungen der Formel I, worin
   Z CO-OR₂ bedeutet, und worin R₂ die in Formel I angegebenen Bedeutungen hat.
(7) Verbindungen der Formel I, worin bedeuten:
   Z CN oder CO-N(R₃)R₄ ;
   R₃ Wasserstoff, C₁-C₅-Alkyl, das unsubstituiert oder mit 1-3 Halogenatomen, C₃-C₆-Cycloalkyl, C₁-C₂-Alkoxy, Phenoxy, Hydroxy, Nitro, Cyano, C₁-C₂-Alkanoyl, Benzoyl, Carboxyl, C₁-C₄-Alkoxycarbonyl, Benzyloxycarbonyl, C₁-C₂-Acyloxy, Benzoyloxy, C₁-C₄-Dialkylamino oder Heterocyclyl substituiert ist, C₃-C₄-Alkenyl, das unsubstituiert oder mit 1-3 Halogenatomen substituiert ist, C₃-C₄-Alkinyl, C₃-C₆-Cycloalkyl, Phenyl, Benzyl oder Phenethyl, deren Phenylringe unsubstitiert oder ein bis zweifach durch Halogen, Hydroxy, C₁-C₄-Alkyl, Halogen-C₁-C₂-alkyl, C₁-C₂-Alkoxy, Halogen-C₁-C₂-alkoxy, oder Nitro substituiert sind, Naphthyl oder Heterocyclyl, das unsubstituiert oder ein- bis zweifach gleich oder verschieden durch Halogen, C₁-C₂-Alkyl, Halogenmethyl oder Nitro substituiert ist;
   R₄ Wasserstoff, C₁-C₄-Alkyl, C₃-C₄-Alkenyl, C₃-C₄-Alkinyl, C₃-C₆-Cycloalkyl, Phenyl oder Benzyl;
   R₃ und R₄ zusammen mit dem Stickstoffatom, an das sie gebunden sind, Pyrrolidin, Piperidin, Morpholin oder Dimethylmorpholin.
(8) Verbindungen der Formel I, worin bedeuten:
   Z CO-N(R₃)R₄;
   R₃ Wasserstoff, C₁-C₅-Alkyl, das unsubstituiert oder mit C₁-C₄-Alkoxycarbonyl oder Benzyloxycarbonyl substituiert ist, C₃-C₄-Alkenyl, C₃-C₄-Alkinyl, C₃-C₆-Cycloalkyl, Phenyl oder Benzyl, deren Phenylringe unsubstitiert oder ein bis zweifach durch Halogen, Hydroxy, Methyl, Methoxy, Halogenmethyl, Halogenmethoxy, oder Nitro substituiert sind;
   R₄ Wasserstoff oder Methyl; oder
   R₃ und R₄ zusammen mit dem Stickstoffatom, an das sie gebunden sind, Pyrrolidin, Piperidin, Morpholin oder Dimethylmorpholin.
(9) Verbindungen der Formel I, worin bedeuten:
   Z CS-A;
   A OR₂, SR₂ oder N(R₃)R₄;
   R₂ und R₃ Wasserstoff, C₁-C₅-Alkyl, das unsubstituiert oder mit 1-3 Halogenatomen, C₃-C₆-Cycloalkyl, C₁-C₂-Alkoxy, Phenoxy, Hydroxy, Nitro, Cyano, C₁-C₂-Alkanoyl, Benzoyl, Carboxyl, C₁-C₄-Alkoxycarbonyl, Benzyloxycarbonyl, C₁-C₂-Acyloxy, Benzoyloxy, C₁-C₄-Dialkylamino oder Heterocyclyl substituiert ist, C₃-C₄-Alkenyl, das unsubstituiert oder mit 1-3 Halogenatomen substituiert ist, C₃-C₄-Alkinyl, C₃-C₆-Cycloalkyl, Phenyl, Benzyl oder Phenethyl, deren Phenylringe unsubstitiert oder ein bis zweifach durch Halogen, Hydroxy, C₁-C₄-Alkyl, Halogen-C₁-C₂-alkyl, C₁-C₂-Alkoxy, Halogen-C₁-C₂-alkoxy, oder Nitro substituiert sind, Naphthyl oder Heterocyclyl, das unsubstituiert oder ein- bis zweifach gleich oder verschieden durch Halogen, C₁-C₂-Alkyl, Halogenmethyl oder Nitro substituiert ist;
   R₄ Wasserstoff, C₁-C₄-Alkyl, C₃-C₄-Alkenyl, C₃-C₄-Alkinyl, C₃-C₆-Cycloalkyl, Phenyl oder Benzyl;
   R₃ und R₄ zusammen mit dem Stickstoffatom, an das sie gebunden sind, Pyrrolidin, Piperidin, Morpholin oder Dimethylmorpholin.
(10) Verbindungen der Formel I, worin bedeuten:
   Z CN, CH=U oder CH₂-V;
   U O, NR₁₀, N-OR₆ oder N-N(=C)ₙ(R₇)R₈;
   V Halogen, Hydroxy, C₁-C₄-Alkoxy, C₁-C₄-Acyloxy, Benzoyloxy, Benzyloxy; und
   worin
   R₆,R₇,R₈ und R₁₀ die in Formel I angegebenen Bedeutungen haben;
   n 0 oder 1.
(11) Verbindungen der Formel I, worin bedeuten:
   Z CH(V)₂;
   V C₁-C₄-Alkoxy oder ein 5-oder 6-gliedriges cyclisches Acetal.
(12) Verbindungen der Formel I, worin bedeuten:
   Z CN, COOH, CHO, CH₂OH, CH₂Cl, CCl₃ CHF₂ oder CF₃.
(13) Verbindungen der Formel Ia, 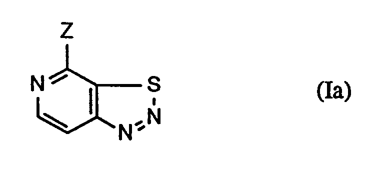 worin Z die in Formel I angegebenen Bedeutungen hat.
(14) Verbindungen der Formel Ib, 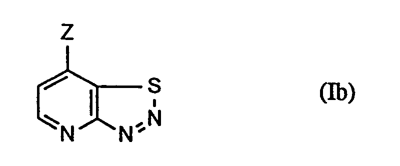 worin Z die in Formel I angegebenen Bedeutungen hat.

Verbindungen der Formel Ia, worin Z CO-N(Alkyl)₂ oder COOH bedeutet, können gemäss Synthese-Schema 1 wie folgt hergestellt werden:
(1) Umsetzung einer Verbindung der Formel VI mit Diethylthiocarbamoylchlorid, (hergestellt nach M. Vilkas, D. Qasmi, Synth. Commun. **20**, 2769, (1990)), in einem inerten, polaren Lösungsmittel wie DMF oder DMPU bei 0-10°C in Gegenwart einer Base wie Triethylamin zu einer Verbindung der Formel VII;
(2) Metallierung einer Verbindung der Formel VII mit einer metallorganischen 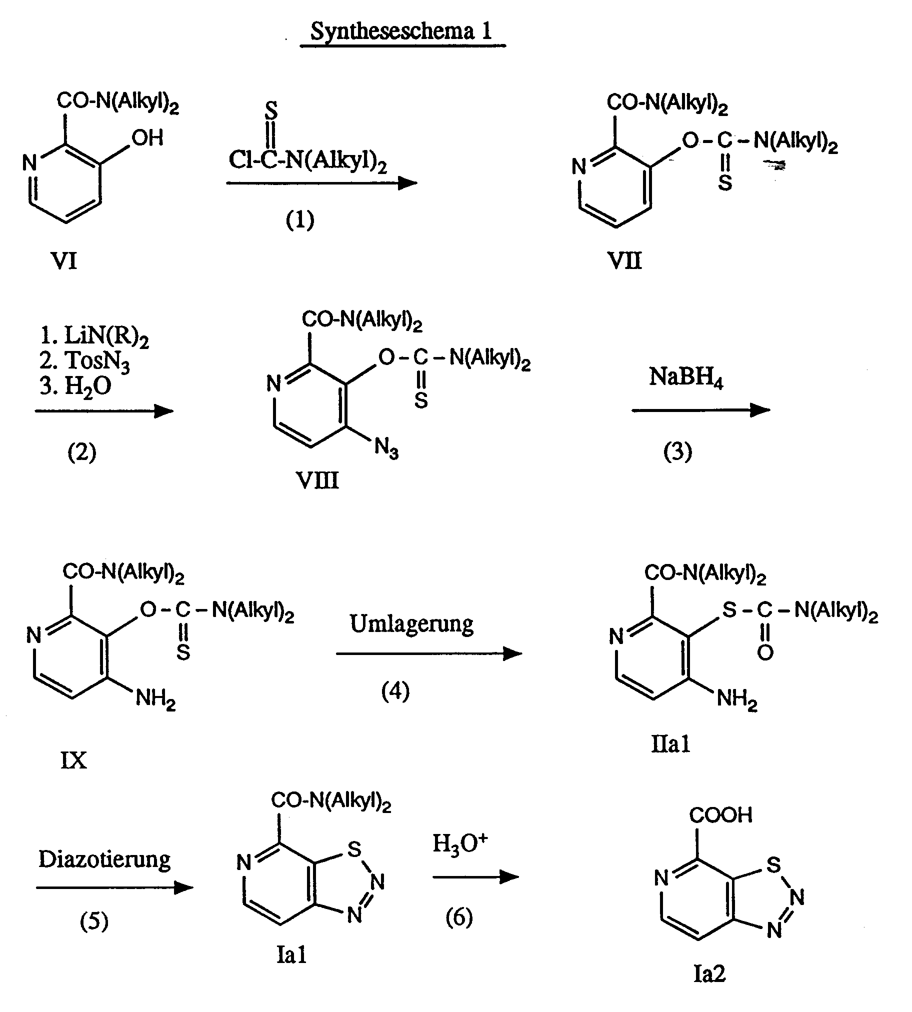 Verbindung wie Lithium-2,2,4,4-tetramethylpiperidid (LTMP) oder Lithiumdiisopropylamid in einem aprotischen Lösungsmittel wie THF oder Hexan bei -100°C bis + 10°C, Umsetzung des gebildeten Metallkomplexes mit Tosylazid (hergestellt nach W. v. Doering, C. H. DePuy, J. Chem. Soc. 5956, (1953) oder Phosphorsäurediphenylesterazid zu einer Verbindung der Formel VIII;
(3/4) Reduktion der Azidogruppe mit einem komplexen Hydrid, z.B. mit NaBH₄ in Methanol bei 0-30°C, wobei direkt die umgelagerte Verbindung der Formel IIa1 entsteht.
(5) Diazotierung einer Verbindung der Formel IIa1, mit einem anorganischen oder organischen Nitrit, z.B. mit Isoamylnitrit in einem inerten Lösungsmittel wie Dimethoxyethan zu einer Verbindung der Formel Ia1.
(6) Gegebenenfalls Hydrolyse der Amidogruppe in Verbindung Ia1 zur Carboxygruppe der Verbindung Ia2.
   Ferner können Verbindungen der Formel Ia, worin Z COOAlkyl oder COOH bedeutet, gemäss Synthese-Schema 2 analog bekannten Verfahren nach B. Blank et al., J. Med. Chem. **17**, 1065, (1974); B. Blank et al., ibid. **20**, 1572, (1977) wie folgt hergestellt werden:
(7) Oxidation einer Verbindung der Formel XI mit Persäure, z.B. m-Chlorperbenzoesäure in Chloroform zum N-Oxid der Formel XII;
(8) Nitrierung des N-Oxides und anschliessend
(9) Umsetzung mit einem Thiol der Formel HS-W oder dessen Salz, worin W einen gegebenenfalls substituierten C₁-C₁₂-Kohlenwasserstoff bedeutet, zu einer Verbindung der Formel XIV;
(10) Reduktion der Nitro- und der N-Oxid-gruppe z.B. mit Eisenpulver in wässriger Säure oder mit Wasserstoff/Katalylastor zu einer Verbindung der Formel IIa2;
(11) Diazotierung einer Verbindung der Formel IIa2, mit einem anorganischen oder organischen Nitrit, z.B. mit Isoamylnitrit, in einem inerten Lösungsmittel wie Dimethoxyethan zu einer Verbindung der Formel Ia3;
   und gegebenenfalls Hydrolyse der Estergruppe in Verbindung Ia3 zur Carboxygruppe der Verbindung Ia2.
   Besonders hervorzuheben ist dabei diejenige Reaktionsstufe, worin Verbindungen der Formel Ia hergestellt werden durch Diazotierung einer Verbindung der Formel IIa 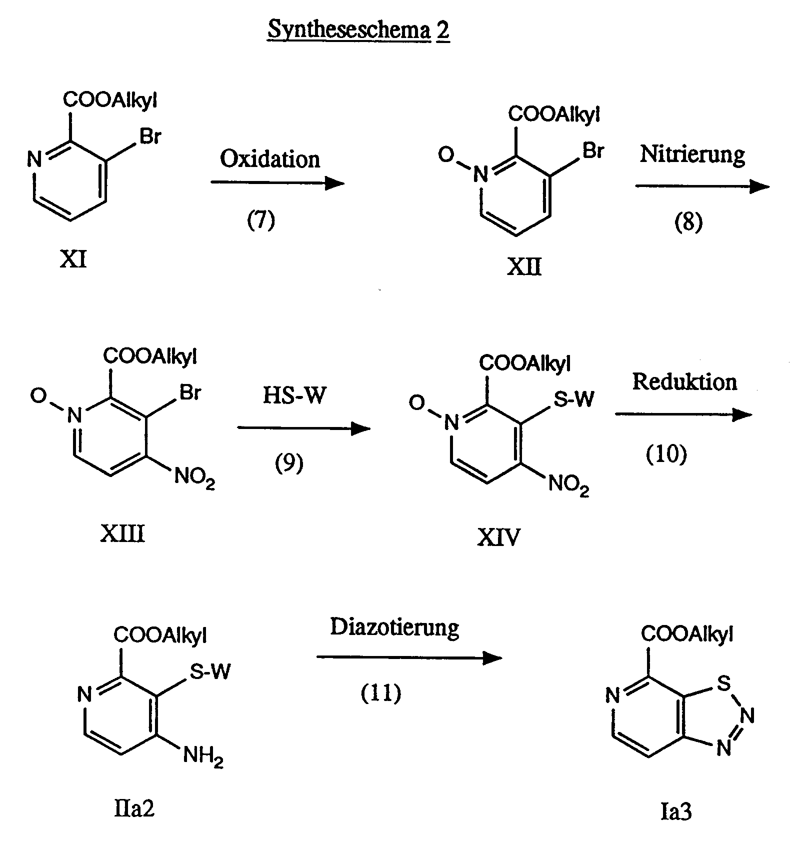 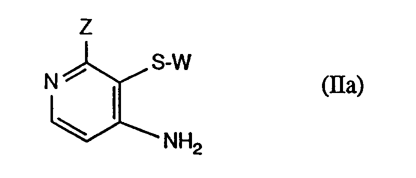 worin Z die für Formel I angegebenen Bedeutungen hat und
   W einen gegebenenfalls substituierten C₁-C₁₂-Kohlenwasserstoff oder eine gegebenenfalls substituierte Carbamoylgruppe bedeutet, mit einem anorganischen oder organischen Nitrit, z.B. Isoamylnitrit, in einem inerten Lösungsmittel, z.B. Dimethoxyethan.
   Verbindungen der Formel Ib, worin Z CO-N(Alkyl)₂ oder COOH bedeutet, können gemäss Synthese-Schema 3 wie folgt hergestellt werden:
(12/13) Metallierung einer Verbindung der Formel XV mit Lithium-2,2,6,6-tetramethylpiperidid (LTMP). Umsetzung mit Borsäuretrimethylester und anschliessender Oxidation z.B. mit H₂O₂, zu einer Verbindung der Formel XVII.
(14) Umsetzung mit Diethylthiocarbamoylchlorid zu einer Verbindung der Formel XVIII.
(15) Metallierung mit LTMP und anschliessender Umsetzung mit Diethylcarbamoylchlorid zu einer Verbindung der Formel XIX.
(16) Umlagerung bei 200°C z.B. in Diphenylether zu einer Verbindung der Formel XX:
(17) Aminolyse mit NH₃ zu einer Verbindung der Formel XXI.
(18) Umsetzung mit Nitrosylschwefelsäure in konzentrierter Schwefelsäure zum entsprechenden Disulfid der Formel XXII.
(19) Diazotierung, mit einem anorganischen oder organischen Nitrit, z.B. Isoamylnitrit, in einem inerten Lösungsmittel, z.B. Dimethoxyethan, zum N-Oxid der Formel XXIII.
(20) Reduktion (z.B. mit Iod/Tris-(dimethylamino)phosphin zu einer Verbindung der Formel Ib1; und gegebenenfalls Hydrolyse der Amidogruppe in Verbindung Ib1 zur Carboxygruppe der Verbindung Ib2.

Besonders hervorzuheben ist dabei diejenige Reaktionsstufe, worin Verbindungen der Formel Ib hergestellt werden durch Reduktion einer Verbindung der Formel XXIII, 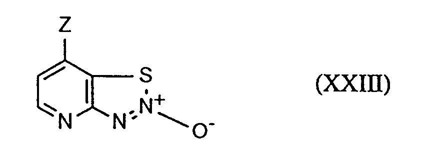 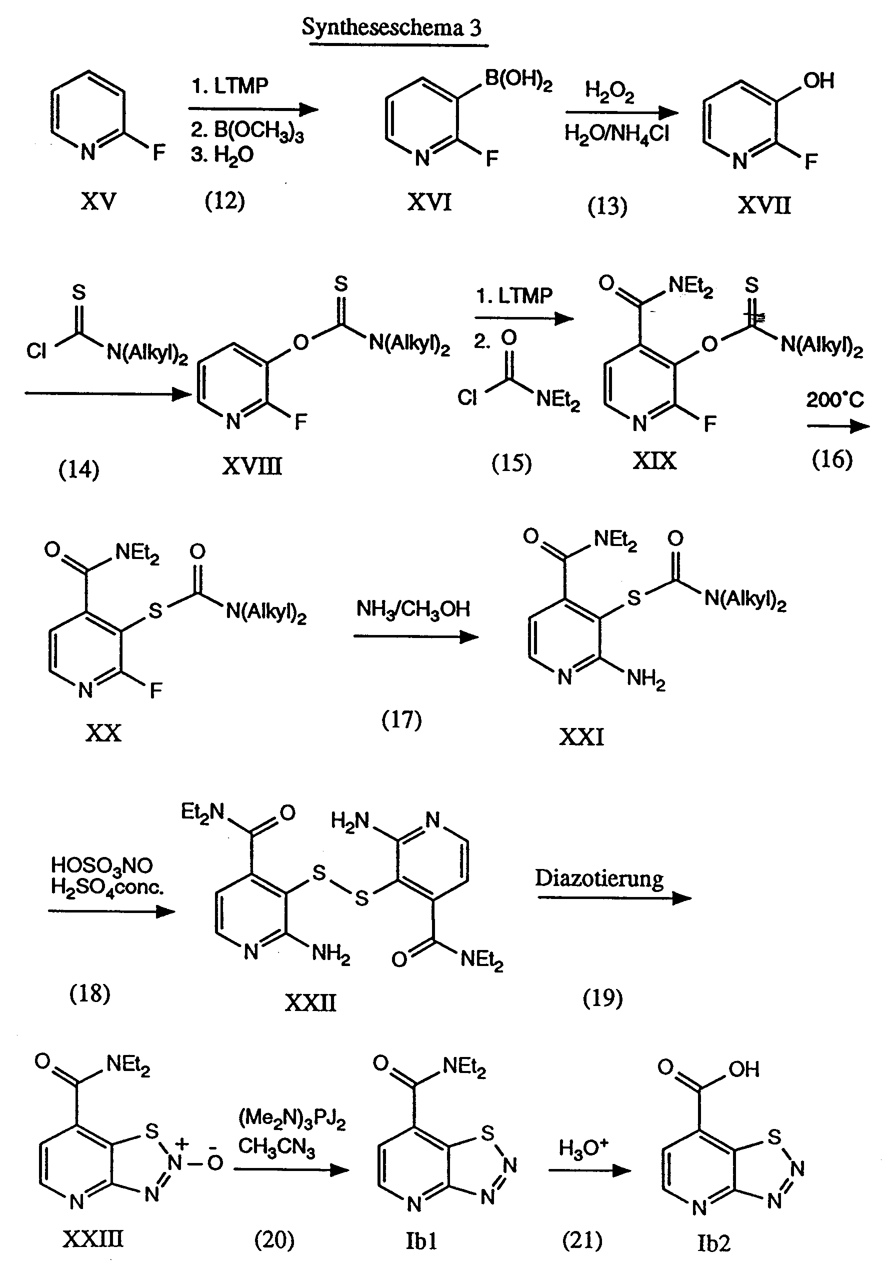
worin Z die für Formel I angegebenen Bedeutungen hat, mit einem Reduktionsmittel, z.B. mit I₂/Tris-(Dimethylamino)phosphin, Hydrazin, Eisen(II)salzen oder Natriumborhydrid.

Verbindungen der Formeln I, worin Z COOH bedeutet, können nach bekannten Methoden in andere Verbindungen der allgemeinen Formel I, worin Z die oben genannten Bedeutungen hat, umgewandelt werden. So können z.B. die Carbonsäuren mit Thionylchlorid in die entsprechenden Carbonsäurechloride übergeführt werden, aus denen sich die Gruppe Z gemäss Sytheseschema 4 wie folgt umwandeln lässt: 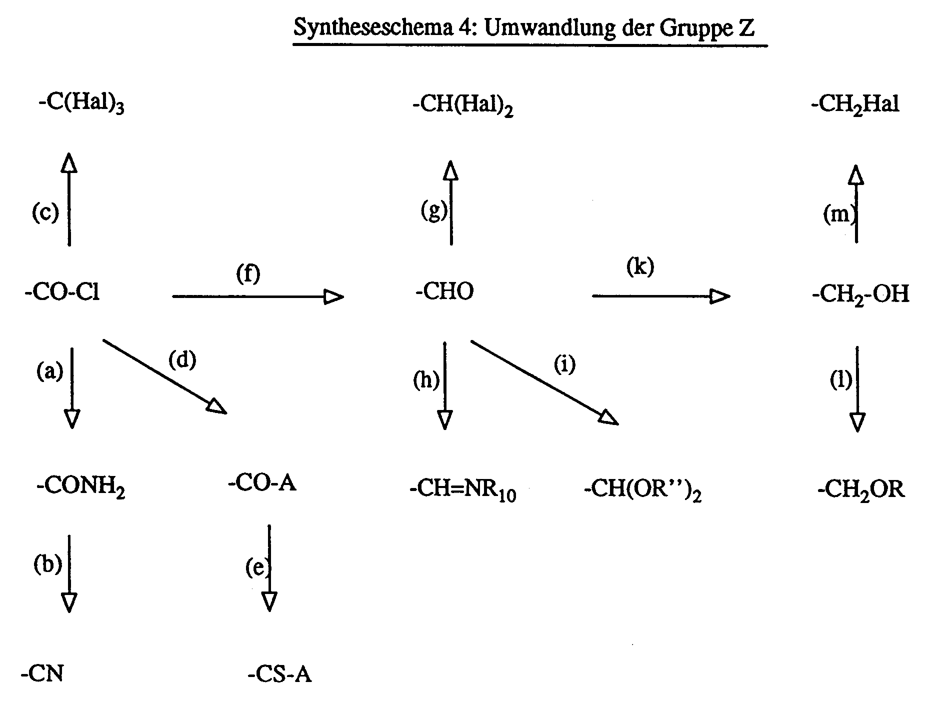
(a) NH₃;
(b) Wasserabspaltungsmittel, z.B. SOCl₂; oder COCl₂;
(c) Halogenierungsmittel, z.B. PCl₅ oder SF₄;
(d) M-A (III), worin M Wasserstoff, Li⁺, Na⁺, K⁺, 1/2Mg²⁺ oder ein quartäres Ammoniumion und A die für Forme I angegebene Bedeutung hat;
(e) Thionierungsmittel, z.B. Phosphorpentasulfid oder 4-Methoxyphenylthiophosphonsäure-cyclodithioanhydrid ("Lawesson-Reagens");
(f) Reduktion, z.B. mit Wasserstoff/Katalysator;
(g) Halogenierungsmittel, z.B. PCl₅ oder SF₄;
(h) H₂NR₁₀, worin R₁₀ die für Formel I angegebene Bedeutung hat;
(i) C₁-C₄-Alkohol oder C₂-C₃-Diol;
(k) Reduktion, z.B. mit Wasserstoff/Katalysator oder mit einem Komplexen Hydrid, z.B. LiAlH₂(OCH₂CH₂OCH₃)₂;
(l) L-R', worin L eine Abgangsgruppe und R' C₁-C₄-Alkyl, C₁-C₄-Acyl, Benzoyl, oder Benzyl bedeuten;
(m) Halogenierungsmittel, z.B. POCl₃, SOCl₂, PBr₃ oder SF₄.

Die beschriebenen Umsetzungen werden in an sich bekannter Weise durchgeführt, z. B. in Ab- oder üblicherweise in Anwesenheit eines geeigneten Lösungs- oder Verdünnungsmittels oder eines Gemisches derselben, wobei man je nach Bedarf unter Kühlen, bei Raumtemperatur oder unter Erwärmen, z. B. in einem Temperaturbereich von etwa -80°C bis zur Siedetemperatur des Reaktionsmediums, vorzugsweise von etwa -20°C bis etwa +150°C, und, falls erforderlich, in einem geschlossenen Gefäss, unter Druck, in einer Inertgasatmosphäre und/oder unter wasserfreien Bedingungen arbeitet.
Reaktionen mit metallorganischen Verbindungen, wie z.B. Lithium-2,2,6,6,-tetramethylpiperidid, werden vorteilhaft bei -100°C bis +10°C durchgeführt.
Diazotierungen, d.h. die Umsetzung eines primären Amins mit salpetriger Säure oder mit einem anorganischen oder organischen Nitrit, werden vorteilhaft bei -20°C bis +30°C durchgeführt.

Abgangsgruppen sind z. B. Fluor, Chlor, Brom, Iod, C₁-C₈-Alkylthio, wie Methylthio, Ethylthio oder Propylthio, C₁-C₈-Alkanoyloxy, wie Acetoxy, (Halogen-)C₁-C₈-Alkansulfonyloxy, wie Methansulfonyloxy, Ethansulfonyloxy oder Trifluormethansulfonyloxy, oder gegebenenfalls substituiertes Phenylsulfonyloxy, wie Benzolsulfonyloxy oder p-Toluolsulfonyloxy, Imidazolyl, Hydroxy oder Wasser.

Geeignete Basen sind z. B. Alkalimetall- oder Erdalkalimetall-hydroxide, -hydride, -amide, -alkanolate, -carbonate, -dialkylamide oder -alkylsilylamide, Alkylamine, Alkylendiamine, gegebenenfalls N-alkylierte, gegebenenfalls ungesättigte Cycloalkylamine, basische Heterocyclen, Ammoniumhydroxide sowie carbocyclische Amine. Beispielhaft seien Natrium-hydroxid, -hydrid, -amid, -methanolat, -carbonat, Kalium-tert.-butanolat, -carbonat, Lithiumdiisopropylamid, Kalium-bis(trimethylsilyl)amid, Calciumhydrid, Triethylamin, Triethylendiamin, Cyclohexylamin, N-Cyclohexyl-N,N-dimethyl-amin, N,N-Diethylanilin, Pyridin, 4-(N,N-Dimethylamino)pyridin, N-Methylmorpholin, Benzyl-trimethyl-ammoniumhydroxid sowie 1,8-Diazabicyclo[5.4.0]undec-5-en (DBU) genannt.

Die Reaktionspartner können als solche, d. h. ohne Zusatz eines Lösungs- oder Verdünnungsmittels, z. B. in der Schmelze, miteinander umgesetzt werden. Zumeist ist jedoch der Zusatz eines inerten Lösungs- oder Verdünnungsmittels oder eines Gemisches derselben vorteilhaft Als Beispiele für solche Lösungs- oder Verdünnungsmittel seien genannt: aromatische, aliphatische und alicyclische Kohlenwasserstoffe und Halogenkohlenwasserstoffe, wie Benzol, Toluol, Xylol, Chlorbenzol, Brombenzol, Petrolether, Hexan, Cyclohexan, Dichlormethan, Trichlormethan, Dichlorethan oder Trichlorethan; Ether, wie Diethylether, tert-Butylmethylether, Tetrahydrofuran oder Dioxan; Ketone, wie Aceton oder Methylethylketon; Alkohole, wie Methanol, Ethanol, Propanol, Butanol, Ethylenglycol oder Glycerin; Ester, wie Essigsäureethylester oder Essigsäurebutylester; Amide, wie N,N-Dimethylformamid, N,N-Dimethylacetamid, N-Methylpyrrolidon oder Hexamethylphosphorsäuretriamid; Nitrile, wie Acetonitril; und Sulfoxide, wie Dimethylsulfoxid. Auch im Ueberschuss eingesetzte Basen, wie Triethylamin, Pyridin, N-Methylmorpholin oder N,N-Diäthylanilin, können als Lösungs- oder Verdünnungsmittel dienen.

Die Umsetzung kann auch unter Phasentransferkatalyse in einem organischen Lösungsmittel, z.B. Methylenchlorid oder Toluol, in Gegenwart einer wässrigen basischen Lösung, z.B. Natriumhydroxid-Lösung, sowie eines Phasentransferkatalysators, z.B. Tetrabutylammoniumhydrogensulfat, erfolgen.

Typische Reaktionsbedingungen können den Beispielen entnommen werden.

Falls die Verbindungen der Formel I in verschiedenen Stereoisomeren auftreten können, betrifft die Erfindung sowohl die reinen Isomeren als auch alle möglichen Isomerengemische.

Neue für die Herstellung der Verbindungen der Formel I verwendete Ausgangs- und Zwischenprodukte, ihre Verwendung und Verfahren zu ihrer Herstellung bilden ebenfalls einen Gegenstand der Erfindung.

Thiadiazolo[4,5-b]pyridine der Formel XXX 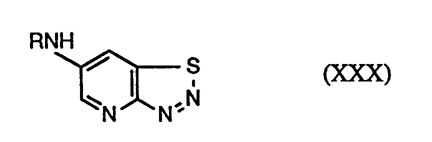
sind in Form von 6-Amino-Derivaten aus der EP-A-321,368 und der EP-A-321,369 zur Verwendung als Süssstoffe bekannt geworden. Es werden in den genannten Referenzen allerdings keine Beispiele und keine Herstellungsmethode dafür angegeben. In J. Org. Chem., Vol.43, 4910-5(1978); und Vol. 41, 3784-8(1976) sind Diamino-thiadiazolo[4,5-c]pyridine der Formel XXXI, 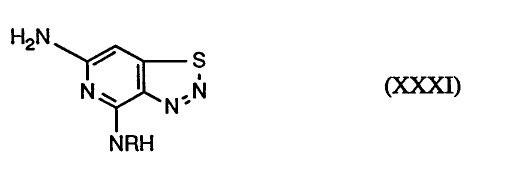
in J. Org. Chem., Vol. 37, 3601-4 (1972) sind Amino-thiadiazolo[4,5-c]pyridine der Formel XXXII und Amino-thiadiazolo[5,4-b]pyridine der Formel XXXIII 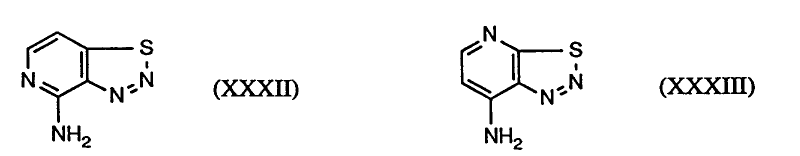
beschrieben, ohne Angaben einer Verwendung für die Verbindungen XXXI, XXXII und XXXIII.

Verbindungen der Formel I unterscheiden sich strukturell in charakteristischer Weise von den obgenannten Verbindungen aus dem Stand der Technik. Sie weisen ferner eine unerwartet hohe mikrobizide Wirksamkeit auf. Dabei kann der Schutz der Pflanzen sowohl durch direkte Einwirkung auf die Schädlinge als auch durch Aktivierung und Stimulierung des pflanzeneigenen Abwehrsystems eintreten (Immunisierung). Durch diese immunisierende Wirkung kann eine Gesunderhaltung und Stärkung der behandelten Pflanzen aus eigener Kraft ohne Einsatz weiterer mikrobizider Substanzen während der Vegetationsperiode gewährleistet werden.

Die Verbindungen der Formel I lassen sich auf dem Agrarsektor und verwandten Gebieten präventiv und/oder kurativ als Wirkstoffe bei der Bekämpfung von Pflanzenschädlingen einsetzen. Die erfindungsgemässen Wirkstoffe der Formel I zeichnen sich durch hervorragende Wirkung auch bei niedrigen Anwendungskonzentrationen, durch gute Pflanzenverträglichkeit und Umweltfreundlichkeit aus. Sie besitzen sehr vorteilhafte, insbesondere systemische, Eigenschaften und können zum Schutz von zahlreichen Kulturpflanzen eingesetzt werden. Mit den Wirkstoffen der Formel I können an Pflanzen oder Pflanzenteilen (Früchten, Blüten, Laubwerk, Stengeln, Knollen, Wurzeln) von unterschiedlichen Nutzkulturen die auftretenden Schädlinge eingedämmt oder vernichtet werden, wobei auch später zuwachsende Pflanzenteile, z. B. von phytopathogenen Mikroorganismen, verschont bleiben.

Die Verbindungen I können ferner als Beizmittel zur Behandlung von Saatgut (Früchten, Knollen, Körnern) und Pflanzenstecklingen zum Schutz vor Pilzinfektionen sowie gegen im Erdboden auftretende phytopathogene Pilze eingesetzt werden.

Die Verbindungen I sind z. B. gegen die den folgenden Klassen angehörenden phytopathogenen Pilze wirksam: Fungi imperfecti (z. B. Botrytis, Pyricularia, Helminthosporium, Fusarium, Septoria, Cercospora und Alternaria) und Basidiomyceten (z. B. Rhizoctonia, Hemileia, Puccinia). Darüber hinaus wirken sie gegen die Klassen der Ascomyceten (z. B. Venturia und Erysiphe, Podosphaera, Monilinia, Uncinula) und der Oomyceten (z. B. Phytophthora, Pythium, Plasmopara).

Als Zielkulturen für den pflanzenschützenden Einsatz gelten im Rahmen der Erfindung beispielsweise folgende Pflanzenarten: Getreide (Weizen, Gerste, Roggen, Hafer, Reis, Mais, Sorghum und verwandte Spezies); Rüben (Zucker- und Futterrüben); Kern-, Stein und Beerenobst (Aepfel, Birnen, Pflaumen, Pfirsiche, Mandeln, Kirschen, Erdbeeren, Himbeeren und Brombeeren); Hülsenfrüchte (Bohnen, Linsen, Erbsen, Soja); Oelkulturen (Raps, Senf, Mohn, Oliven, Sonnenblumen, Kokos, Rizinus, Kakao, Erdnüsse); Gurkengewächse (Kürbis, Gurken, Melonen); Fasergewächse (Baumwolle, Flachs, Hanf, Jute); Citrusfrüchte (Orangen, Zitronen, Pampelmusen, Mandarinen); Gemüsesorten (Spinat, Kopfsalat, Spargel, Kohlarten, Möhren, Zwiebeln, Tomaten, Kartoffeln, Paprika); Lorbeergewächse (Avocado, Cinnamonium, Campfer) und Pflanzen wie Tabak, Nüsse, Kaffee, Eierfrüchte, Zuckerrohr, Tee, Pfeffer, Reben, Hopfen, Bananen- und Naturkautschukgewächse sowie Zierpflanzen.

Wirkstoffe I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können z. B. Düngemittel, Spurenelemente-Vermittler oder andere das Pflanzenwachstum beeinflussende Präparate sein. Es können dabei auch selektive Herbizide sowie Insektizide, Fungizide, Bakterizide, Nematizide, Molluskizide oder Gemische mehrerer dieser Präparate zusammen mit gegebenenfalls weiteren in der Formulierungstechnik üblichen Trägerstoffen, Tensiden oder anderen applikationsfördernden Zusätzen Verwendung finden.

Geeignete Träger und Zusätze können fest oder flüssig sein und sind in der Formulierungstechnik zweckdienliche Stoffe, z. B. natürliche oder regenerierte mineralische Stoffe, Lösungs-, Dispergier-, Netz-, Haft-, Verdickungs-, Binde- oder Düngemittel.

Ein bevorzugtes Verfahren zum Aufbringen eines Wirkstoffs der Formel I bzw. eines agrochemischen Mittels, das mindestens einen dieser Wirkstoffe enthält, ist das Aufbringen auf das Blattwerk (Blattapplikation). Applikationsfrequenz und Aufwandmenge richten sich dabei nach dem Befallsdruck des betreffenden Erregers. Die Wirkstoffe I können aber auch über den Erdboden durch das Wurzelwerk in die Pflanze gelangen (systemische Wirkung), indem man den Standort der Pflanze mit einer flüssigen Zubereitung tränkt oder die Substanzen in fester Form in den Boden einbringt, z. B. in Form von Granulat (Bodenapplikation). Bei Wasserreiskulturen kann man solche Granulate dem überfluteten Reisfeld zudosieren. Die Verbindungen I können aber auch zur Saatgutbehandlung auf Samenkörner aufgebracht werden (Coating), indem man die Körner bzw. Knollen entweder in einer flüssigen Zubereitung des Wirkstoffs tränkt oder sie mit einer festen Zubereitung beschichtet.

Die Verbindungen I werden dabei in unveränderter Form oder vorzugsweise zusammen mit den in der Formulierungstechnik üblichen Hilfsmitteln eingesetzt. Dafür werden sie zweckmässigerweise z. B. zu Emulsionskonzentraten, streichfählgen Pasten, direkt versprühbaren oder verdünnbaren Lösungen, verdünnten Emulsionen, Spritzpulvern, löslichen Pulvern, Stäubemitteln oder Granulaten, z. B. durch Verkapselung in, z. B. polymeren, Stoffen, in bekannter Weise verarbeitet. Die Anwendungsverfahren wie Versprühen, Vernebeln, Verstäuben, Verstreuen, Bestreichen oder Giessen werden ebenso wie die Art der Mittel den angestrebten Zielen und den gegebenen Verhältrissen entsprechend gewählt.

Günstige Aufwandmengen liegen im allgemeinen bei 5 g bis 2 kg Aktivsubstanz (AS) je Hektar (ha), bevorzugt bei 10 g bis 1 kg AS/ha, insbesondere bei 20 g bis 600 g AS/ha. Bei der Verwendung als Saatbeizmittel werden mit Vorteil Dosierungen von 10 mg bis 1 g Aktivsubstanz pro kg Saatgut verwendet.

Die Formulierungen, d. h. die den Wirkstoff der Formel I und gegebenenfalls einen festen oder flüssigen Zusatzstoff enthaltenden Mittel, Zubereitungen oder Zusammensetzungen, werden in bekannter Weise hergestellt, z. B. durch inniges Vermischen und/oder Vermahlen des Wirkstoffs mit Streckmitteln, wie Lösungsmitteln, festen Trägerstoffen und gegebenenfalls oberflächenaktiven Verbindungen (Tensiden).

Als Lösungsmittel kommen in Frage: Aromatische Kohlenwasserstoffe, bevorzugt die Fraktionen C₈ bis C₁₂, wie Xylolgemische oder substituierte Naphthaline, Phthalsäureester, wie Dibutyl- oder Dioctylphthalat, aliphatische Kohlenwasserstoffe, wie Cyclohexan oder Paraffine, Alkohole und Glycole sowie deren Ether und Ester, wie Ethanol, Ethylenglycol, Ethylenglycolmonomethyl- oder -ethylether, Ketone, wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, und Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen, wie Bimsstein, Ziegelbruch, Sepiolit oder Bentonit, als nichtsorptive Trägermaterialien z. B. Calcit oder Sand in Frage. Darüber hinaus kann eine Vielzaal von vorgranulierten Materialien anorganischer oder organischer Natur, wie Dolomit oder zerkleinerte Pflanzenrückstände, verwendet werden.

Als oberflächenaktive Verbindungen kommen je nach Art des zu formulierenden Wirkstoffs der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sogenannte wasserlösliche Seifen, als auch wasserlösliche synthetische oberflächenaktive Verbindungen sein.

Als Beispiele nichtionischer Tenside seien Nonylphenolpolyethoxyethanole, Ricinusölpolyglycolether, Polypropylen-/Polyethylen-oxidaddukte, Tributylphenoxypolyethylenethanol, Polyethylenglycol und Octylphenoxypolyethoxyethanol erwähnt. Ferner kommen auch Fettsäureester von Polyoxyethylensorbitan, wie das Polyoxyethylensorbitan-trioleat, in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen.

Weitere in der Formulierungstechnik gebräuchlichen Tenside sind dem Fachmann bekannt oder können der einschlägigen Fachliteratur entnommen werden.

Die agrochemischen Zubereitungen enthalten in der Regel 0,1 bis 99 Gewichtsprozent, insbesondere 0,1 bis 95 Gewichtsprozent, Wirkstoff der Formel I, 99,9 bis 1 Gewichtsprozent, insbesondere 99,8 bis 5 Gewichtsprozent, eines festen oder flüssigen Zusatzstoffes und 0 bis 25 Gewichtsprozent, insbesondere 0,1 bis 25 Gewichtsprozent, eines Tensids.

Während als Handelsware eher konzentrierte Mittel bevorzugt werden, verwendet der Endverbraucher in der Regel verdünnte Mittel.

Die Mittel können auch weitere Zusätze, wie Stabilisatoren, Entschäumer, Viskositätsregulatoren, Bindemittel oder Haftmittel, sowie Dünger oder andere Wirkstoffe zur Erzielung spezieller Effekte enthalten.

### 1. Herstellungsbeispiele

Temperaturen sind in Celsiusgraden angegeben. Die folgenden Abkürzungen werden verwendet: Ac = Acetyl; Et = Ethyl; i-Pr = Isopropyl; Me = Methyl; Ph = Phenyl; Pr = n-Propyl; Bu = n-Butyl; THF = Tetrahydrofuran; Et₂O = Diethylether; TMP = Tetramethylpiperidin; LTMP = Lithium-tetramethylpiperidin; RT = Raumtemperatur; RG = Reaktionsgemisch; i.V. = im Vacuum; AcOH = Essigsäure; DMF = Dimethylformamid; Smp. = Schmelzpunkt. "NMR" steht für "Kernmagnetisches Resonanzspektrum". MS = Maasenspektrum. "%" steht für "Gewichtsprozent", sofern entsprechende Konzentrationen nicht in einer anderen Einheit angegeben sind.

### Beispiel H-1

### Herstellung von 3-N,N-Diethylthiocarbamoyloxy-picolinsäurediethylamid:

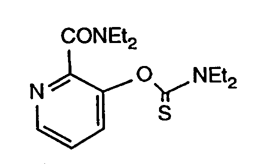

258.3g (1.33mol) 3-Hydroxypicolinsäurediethylamid werden in 1 l
Dimethylpropylenharnstoff und 213.2 ml (1.53 mol) Triethylamin bei 0°C vorgelegt und 222g (1.464 mol) Diethylthiocarbamoylchlorid als Festsubstanz eingetragen. Nach 2 Std. rühren bei 0°C wird das Reaktionsgemisch auf 2 l Wasser gegossen, dreimal mit Essigester extrahiert, fünfmal mit je 500 ml Wasser gewaschen, die vereinigten org. Phasen über Na₂SO₄ getrocknet und im Vakuum bei 60°C eingeengt. Umkristallisation aus Et₂O/Hexan ergeben 341g (83%) braune Kristalle, Smp. 70-72°C.

### Beispiel H-2

### Herstellung von 4-Azido-3-N,N-diethylthiocarbamoyloxy-picolinsäurediethylamid:

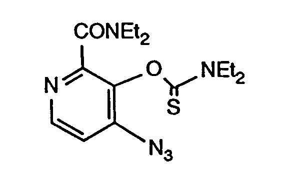

Zu einer Lösung von 88.2g (0.624mol) 2,2,6,6-Tetramethylpiperidin in 1 l THF werden bei -20°C unter Argon 378 ml n-Butyllithium (1.6M in Hexan) gegeben und 1 Std. bei dieser Temperatur gerührt. Danach wird das Gemisch auf -100°C gekühlt und eine Lösung von 104.4g (0.337mol) 3-N,N-diethylthiocarbamoyloxy-picolinsäurediethylamid in 300 ml THF so zugetropft, dass die Temperatur nicht über -90°C steigt Nach 1 Std. Rühren bei -95° C wird erneut auf -102° C gekühlt und eine auf -20° C gekühlte Lösung von 119.3g (0.605 mol) Tosylazid in 200 ml THF innert 30 sec. dazugegeben. Nach 15 Min. rühren bei -80° C werden 400 ml einer gesättigten NH₄Cl-Lösung dazugegeben, auf 0° C erwärmt, die wässrige Phase abgetrennt und mit 500 ml Diethylether gewaschen. Die org. Phasen werden einmal mit 200 ml gesättigter NH₄Cl-Lösung, zweimal mit je 150 ml gesättigter NaCl-Lösung gewaschen, über Na₂SO₄ getrocknet und i.V. eingeengt. Das braune Oel wird in 1.2 l Hexan heiss aufgenommen und unter Rühren langsam auf 0° C abgekühlt, der Festkörper abfiltriert und getrocknet.
Ausbeute: 109.0 g beigebraune Kristalle, die direkt in die nächste Stufe eingesetzt werden.

### Beispiel H-3

### Herstellung von 4-Amino-3-N,N-diethylcarbamoylthio-picolinsäurediethylamid:

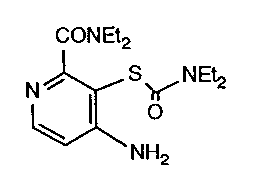

Zu einer Lösung von 191.0g (0.545 mol) 4-Azido-3-N,N-diethylthiocarbamoyloxy-picolinsäurediethylamid in 1 l Methanol werden bei 8° C innerhalb von 2 Std. 25.0g (0.66 mol) NaBH₄ in Portionen so eingetragen, dass die Temperatur 15° C nicht übersteigt. Nach 30 Min. Rühren wird das Reaktionsgemisch auf 8° C gekühlt und mit ca. 200 ml 3N HCl auf pH 7 gestellt und der Methylalkohol bei 60° C i.V. weitgehend abdestilliert. Der Rückstand wird in 500 ml Essigester aufgenommen, 3x mit Wasser gewaschen, die org. Phase über Na₂SO₄ getrocknet und bei 60° C i.V. eingeengt. Der kristalline Rückstand wird aus Essigester/Diethylether/Hexan umkristallisiert.
Ausbeute: 141g (80%) farblose Kristalle, Smp. 144-147°C.

### Beispiel H-4

### Herstellung von 7-[1,2,3]Thiadiazol[5,4-c]-pyridincarbonsäurediethylamid:

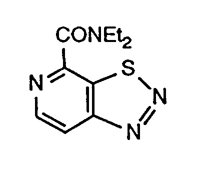

Zu einer Lösung von 161.3g (0.497 mol) 4-Amino-3-N,N-diethylcarbamoylthio-picolinsäurediethylamid in 3 l Dimethoxyethan (DME) werden bei -25° C 420 ml Essigsäure und anschliessend 120 ml Trifluoressigsäure (TFA) eingetragen. Anschliessend werden bei -25 bis -20° C 160 ml (1.2 mol) Isoamylnitrit innert 10 Min. zugetropft und das Reaktionsgemisch über Nacht gerührt, wobei die Temperatur von 0° C auf 12° C steigt. Das Reaktionsgemisch wird bei 60° C i.V. eingeengt, der Rückstand in 1.5 l Essigester und 2 l Wasser aufgenommen, mit ca 90g NaHCO₃ auf pH 7 gestellt, die wässrige Phase abgetrennt, 2x mit Essigester gewaschen, die vereinigten org. Phasen 2x mit Wasser gewaschen, über Na₂SO₄ getrocknet und bei 60° C i.V. eingeengt. Der Rückstand wird in Diethylether aufgenommen, durch 500g Kieselgel filtriert und aus Diethylether/Hexan umkristallisiert.
Ausbeute: 100g (85%) farblose Kristalle, Smp. 97-99° C.

### Beispiel H-5

### Herstellung von 7-[1,2,3]Thiadiazol[5,4-c]-pyridincarbonsäure:

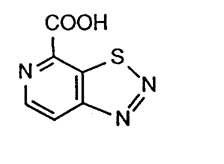

100g (0.423 mol) 7-[1,2,3]Thiadiazol[4,5-c]-pyridincarbonsäurediethylamid werden in 500 ml Essigsäure und 500 ml conc. Salzsäure 5 Std. am Rückfluss gekocht. Danach wird die Essigsäure bei 60° C i.V. entfernt, der Rückstand mit 1.5 l Wasser versetzt, auf 0° C gekühlt, die gelblichen Kristalle abgenutscht, mit Eiswasser gewaschen und bei 60° C i.V. getrocknet.
Ausbeute: 70g (91%) gelbliche Kristalle, Smp. 174-175° C (Zers.).

### Beispiel H-6

### Herstellung von 7-[1,2,3]Thiadiazol[5,4-c]-pyridincarbonsäuremethylester:

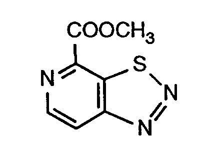

Zu einer Suspension von 27g (0.149 mol) 7-[1,2,3]Thiadiazol[4,5-c]-pyridincarbonsäure in 250 ml Benzol werden bei ca. 25° C 32 ml (0.441 mol) Thionylchlorid eingetragen, auf Rückfluss erhitzt und 0.1 ml Dimethylformamid (DMF) zugefügt. Nach 2 Std. Rückfluss werden erneut 16 ml Thionylchlorid dazugegeben und eine weitere Std. am Rückfluss gekocht. Danach wird das Reaktionsgemisch bei 60° C i.V. eingeengt; man erhält 29.9 g violette Kristalle. Diese werden in 200 ml Methanol aufgenommen, es werden 13.3 ml Pyridin zugegeben und 2 Std. gerührt. Das Reaktionsgemisch wird bei 60°C i.V. eingedampft, der Rückstand in Essigester aufgenommen, mit Wasser gewaschen, wobei das Produkt teilweise ausfällt. Nach Erwärmen auf 60° C wird die klare Lösung mit Aktivkohle behandelt, nach Filtration bei 60° C i.V. eingeengt und der Rückstand aus Diethylether umkristallisiert.
Ausbeute: 26.6g (92%) rosa Kristalle, Smp. 180-182° C.

### Beispiel H-7

### Herstellung von 2-Fluor-3-dihydroxyboryl-pyridin

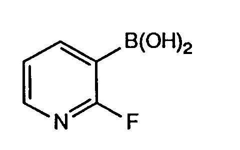

Zu einer Lösung von 188 ml (1.32 mol) TMP in 1000 ml THF werden unter Argon bei -78°C 800 ml (1.28 mol) n-BuLi (1.6 M in Hexan) hinzugefügt. Nach 45 Min. Rühren bei dieser Temperatur werden 120.4 g (1.24 mol) 2-Fluorpyridin in 200 ml THF innert 3 Min. dazugegeben. Nach 4 Std. Rühren bei -78°C wird eine Lösung von 128.8 g (1.24 mol) Trimethylborat in 200 ml THF innert 20 Min. zugetropft. Danach werden bei 25°C 200 ml eines 1.1 Gemisches von Wasser/THF zugegeben und anschliessend unter Eiskühlung 256 ml HCl conc. zugetropft. Die organische Phase wird abgetrennt, die Wasserphase 2 mal mit Et₂O gewaschen, filtriert und im Vakuum eingeengt. Rohausbeute 148.2 g.

### Beispiel H-8

### Herstellung von 2-Fluor-3-hydroxy-pyridin

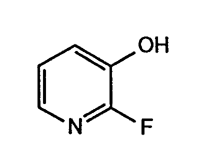

Zu einer Lösung von 61.1 g (434 mmol) 2-Fluor-3-dihydroxyboryl-pyridin in 500 ml Wasser werden bei Raumtemperatur 30 g (560 mmol) NH₄Cl zugefügt. Danach werden innerhalb 30 Min. 300 ml ein 30 %igen H₂O₂-Lösung zugetropft und die Temperatur mittels Eiskühlung unter 25°C gehalten; über Nacht wird bei Raumtemperatur gerührt, danach auf 0°C gekühlt, der Festkörper abgenutscht und die wässrige Phase noch 3 mal mit Ethylacetat extrahiert, die vereinigten organischen Phasen über Na₂SO₄ getrocknet und im Vakuum bei 60°C eingeengt. Ausbeute 44.5 g farblose Kristalle vom
Smp. 131-133°C.

### Beispiel H-9

### Herstellung von 3-Diethylthiocarbamoyloxy-2-fluorpyridin

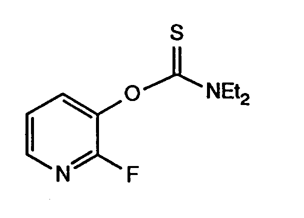

Zu einer Suspension von 36.7 g (325 mmol) 2-Fluor-3-hydroxypyridin in 150 ml Toluol werden bei Raumtemperatur nacheinander 36.2 g (358 mmol) Triethylamin und 54.3 g (358 mmol) Diethylthiocarbamoylchlorid zugefügt und das RG auf 75°C erwärmt. Nach 1 Std. Rühren bei dieser Temperatur wird das RG auf Raumtemperatur gekühlt, mit Ethylacetat verdünnt, 3 mal mit Wasser gewaschen, über Na₂SO₄ getrocknet und im Vakuum bei 60°C eingeengt; 80 g braunes Oel, welches nach Umkristallisation aus Et₂O/Hexan 64.2 g (86 %) braune Kristalle vom Smp. 52-54°C ergab.

### Beispiel H-10

### Herstellung von 3-Diethylthiocarbamoyloxy-2-fluor-nicotinsäurediethylamid

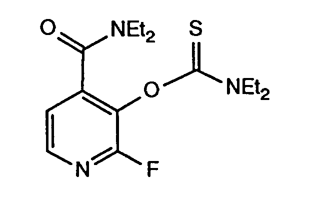

Zu einer Lösung von 57.9 g (410 mmol) TMP in 750 ml THF werden bei -10°C 250 ml (400 mmol) n-BuLi (1.6 M in Hexan) zugefügt. Nach 1 Std. Rühren bei -10°C wird bei -78°C eine Lösung von 64 g (280 mmol) 3-Diethylthiocarbamoyloxy-2-fluor-pyridin in 200 ml THF innert 30 Min. zugetropft. Nach 1 Std. Rühren bei -78°C werden 81,4 g (600 mmol) Diethylcarbamoylchlorid innert 1 Min. zugefügt, 2 Std. bei -78°C gerührt und auf -20°C erwärmt, 130 ml 3N HCl zugefügt, mit 1 l Wasser verdünnt und auf pH 7 gestellt. Die organische Phase wird 3 mal mit Wasser gewaschen. Die vereinigten Wasserphasen werden 2 mal mit Et₂O extrahiert, die vereinigten organischen Phasen 2 mal mit Wasser gewaschen, über Na₂SO₄ getrocknet und im Vakuum bei 60°C eingeengt: 116 g braunes Oel. Chromatographie (Ethylacetat/Hexan 1:1) ergibt 60 g (65 %) beige Kristalle vom Smp. 87-92°C.

### Beispiel H-11

### Herstellung von 3-Diethylcarbamoylthio-2-fluor-nicotinsäurediethylamid

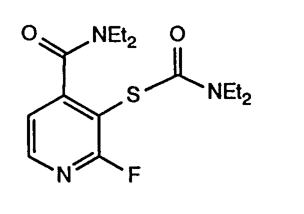

60 g (183 mmol) 3-Diethylthiocarbamoyloxy-2-fluor-nicotinsäurediethylamid werden in 500 g Diphenylether unter Argon während 8 Std. auf 200°C erwärmt. Danach wird der Diphenylether bei 120°C/0.05 bar abdestilliert und der Rückstand an Kieselgel mit Et₂O chromatngraphiert. Nach anschliessender Umkristallisation werden 40.5 g (68 %) beige Kristalle vom Smp. 87-88°C erhalten.

### Beispiel H-12

### Herstellung von 2-Amino-3-diethylcarbamoylthio-nicotinsäurediethylamid

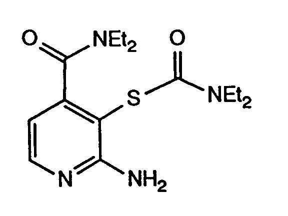

In einem Autoklaven werden auf eine Lösung von 40.2 g (122 mmol) 3-Diethylcarbamoylthio-2-fluor-nicotinsäurediethylamid in 350 g Methanol 43 g Ammoniak aufgepresst und während 10 Std. auf 100°C erhitzt. Danach wird im Vakuum eingeengt und an Kieselgel mit Ethylacetat chromatographiert. Anschliessende Umkristallisation aus Et₂O/Hexan ergibt 18.7 g (47 %) gelbliche Kristalle.

### Beispiel H-13

### Herstellung von Di-(2-amino)-nicotinsäurediethylamido-3,3'-disulfid

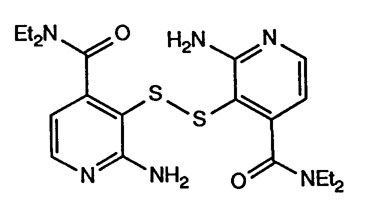

Zu einer Lösung von 18.0 g (55.0 mmol) 2-Amino-3-diethylcarbamoylthio-nicotinsäurediethylamid in konzentrierter Schwefelsäure werden 8.4 g (66 mmol)Nitrosylschwefelsäure gegeben und 2 Std. bei 50°C gerührt. Danach wird das Gemisch bei Raumtemperatur auf eine Lösung von 96 g NaOH in 550 ml Eiswasser gegossen, mit NaHCO₃ auf pH 7-8 gestellt, mit NaCl gesättigt und 6 mal mit Ethylacetat extrahiert. Die vereingten organischen Extrakte werden über Na₂SO₄ getrocknet und im Vakuum bei 60°C eingeengt. Umkristallisation des braunen Harzes aus Ethylacetat/Hexan ergibt 13.9 g (47 %) gelbe Kristalle vom Smp. 176/179°C.

### Beispiel H-14

### Herstellung von 7-[1,2,3]Thiadiazol[4,5-b]-pyridincarbonsäurediethylamid-N-oxid

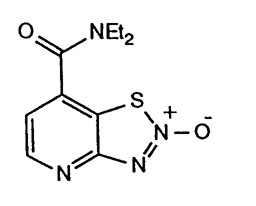

Zu einer Lösung von 13.8 g (30.8 mmol) Di-(2-amino-nicotinsäurediethylamido)-3,3'-disulfid in 200 ml Dimethoxyethan (DME) werden bei Raumtemperatur 10.5 g (92 mmol) Trifluoressigsäure zugefügt, auf 50°C erwärmt und eine Lösung von 8.67 g (74 mmol) Isoamylnitrit in 50 ml DME innerhalb von 1 Std. zugetropft. Nach 30 Min. Rühren bei 50°C wird das RG auf 0°C gekühlt und ein Impfkristall dazugegeben und anschliessend filtriert. Nach Waschen des Festkörpers werden 4 g (26 %) beige Kristalle erhalten.

### Beispiel H-15

### Herstellung von 7-[1,2,3]Thiadiazol[4,5-b]-pyridincarbonsäurediethylamid

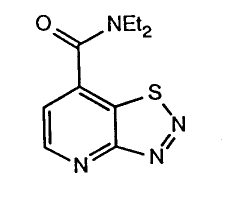

Zu einer Lösung von 4.57 g (18 mmol) Jod in 45 ml Acetonitril wird innerhalb 15 Min. eine Lösung von 2.94 g (18 mmol) Tris-(dimethylamino)-phosphin getropft. Danach werden 3.0 g (11.9 mmol) 7-[1,2,3]Thiadiazol[4,5-b]-pyridincarbonsäurediethylamid-N-oxid zugefügt und 10 Std. am Rückfluss gekocht Nach Abkühlen auf Raumtemperatur wird auf eine verd. Natriumthiosulfat-Lösung gegossen und 3 mal mit Ethylacetat extrahiert. Die vereinigten organischen Phasen werden über Na₂SO₄ getrocknet und im Vakuum bei 60°C eingeengt. Nach Chromatographie mit Et₂O an Kieselgel werden 1.8 g (66 %) gelbliche Kristalle von Smp. 186-188°C erhalten.

### Beispiel H-16

### Herstellung von 7-[1,2,3]Thiadiazol[4,5-b]-pyridincarbonsäure

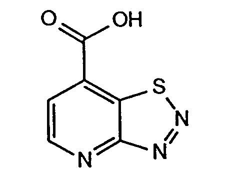

2.3 g (9.73 mmol) 7-[1,2,3]Thiadiazol[4,5-b]-pyridincarbonsäurediethylamid werden in einem Gemisch von 10 ml AcOH und 10 ml HCl conc. 5 Std. am Rückfluss gekocht. Danach wird nicht ganz zur Trockne im Vakuum bei 60°C eingeengt, in 75 ml H₂O aufgeschlämmt, auf 0°C abekühlt, der Festkörper abgenutscht und im Vakuum bei 60°C getrocknet: 1.45 g (82 %) hellbraunes Pulver vom Smp. 230°C (Zers.).

### Beispiel H-17

### Herstellung von 7-[1,2,3]Thiadiazol[4,5-b]-pyridincarbonsäuremethylester

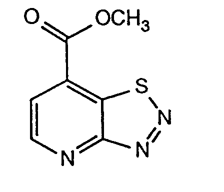

Zu einer Suspension von 0.57 g (3.1 mmol) 7-[1,2,3]Thiadiazol[4,5-b]-pyridincarbonsäure in 20 ml Benzol werden 0.93 g (7.9 mmol) Thionylchlorid und 1 Tropfen DMF zugefügt. Nach 1 Std. Kochen am Rückfluss wird erneut 0.93 g (7.8 mmol) Thionylchlorid eingetragen und weitere 2.5 Std. am Rückfluss gekocht. Danach wird im Vakuum bei 60°C eingeengt und in 50 ml Methanol aufgenommen und solange gerührt, bis eine klare Lösung entsteht. Darauf wird im Vakuum bei 60°C eingeengt, der Rückstand in Ethylacetat aufgenommen, an 25 g Kieselgel filtriert und erneut eingeengt und getrocknet: 0.6 g (99 %) gelbliche Kristalle vom Smp. 145-147°C.

In analoger Weise wie in den vorgenannten Beispielen beschrieben oder nach einer anderen der vorstehend angegebenen Methoden lassen sich die in den folgenden Tabellen aufgeführten Verbindungen herstellen:

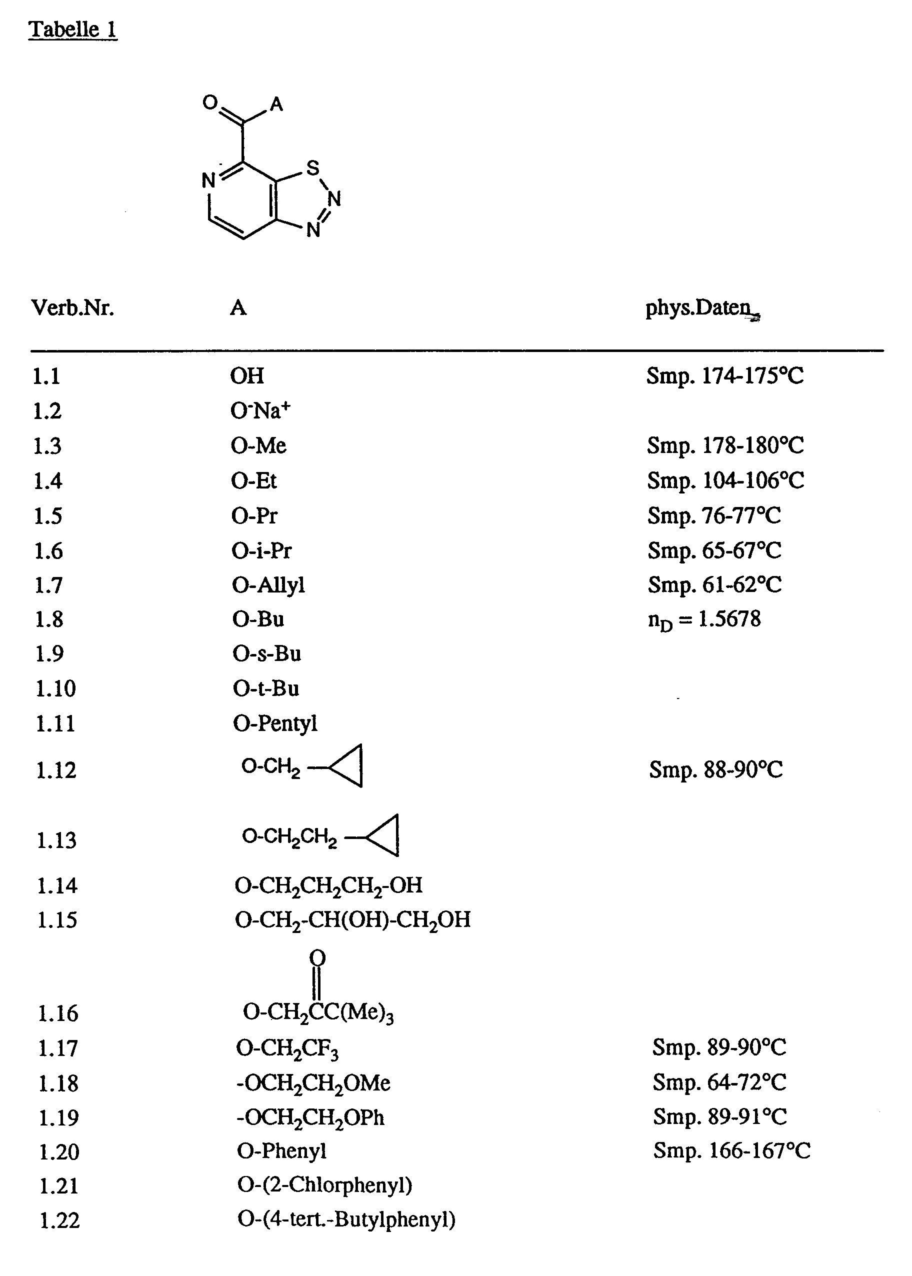

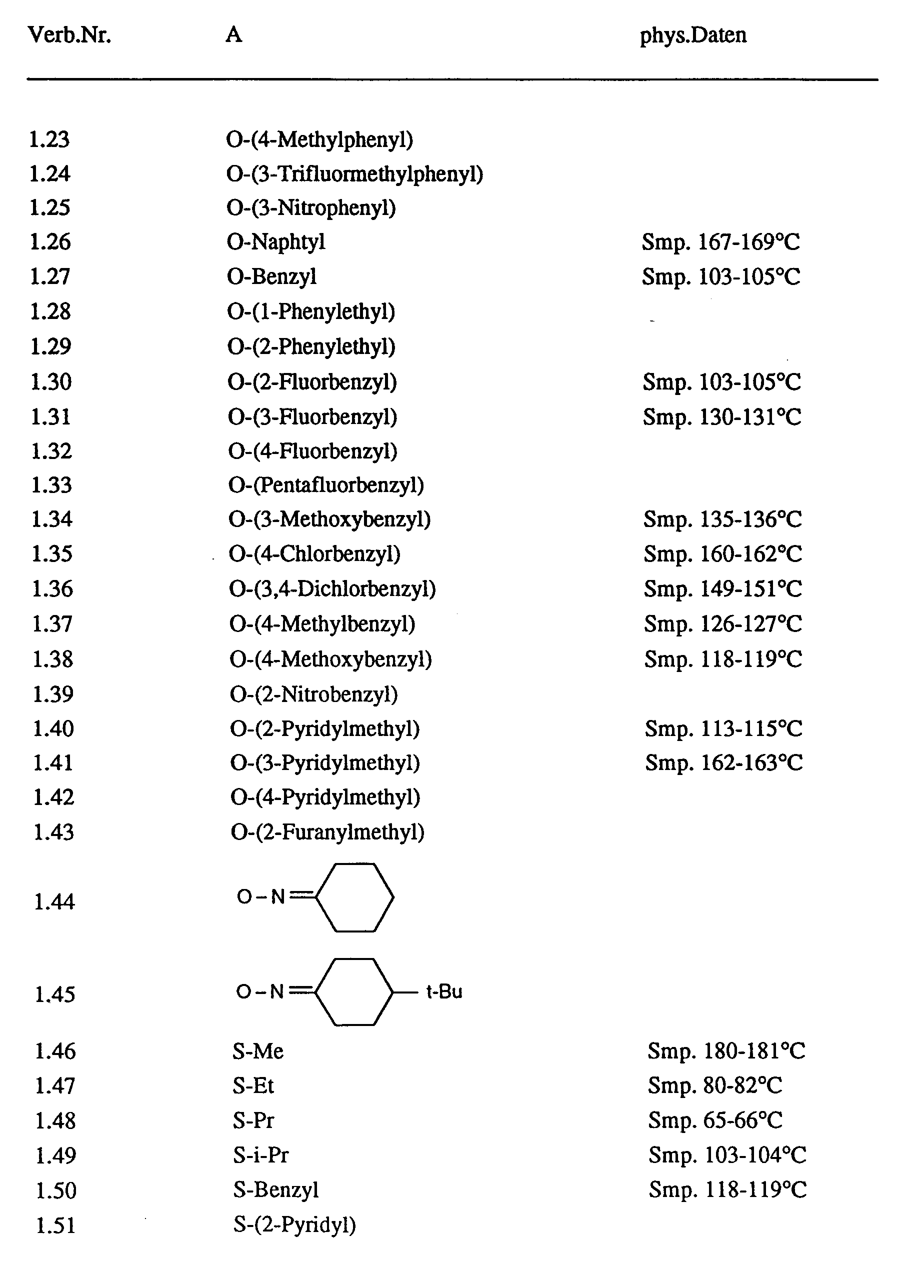

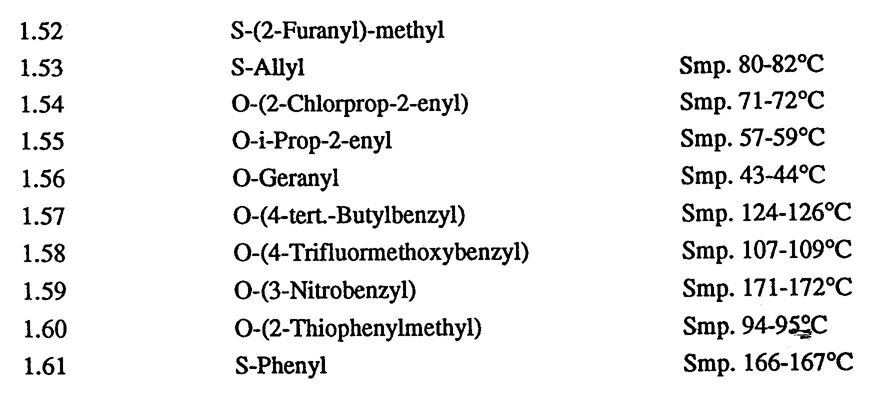

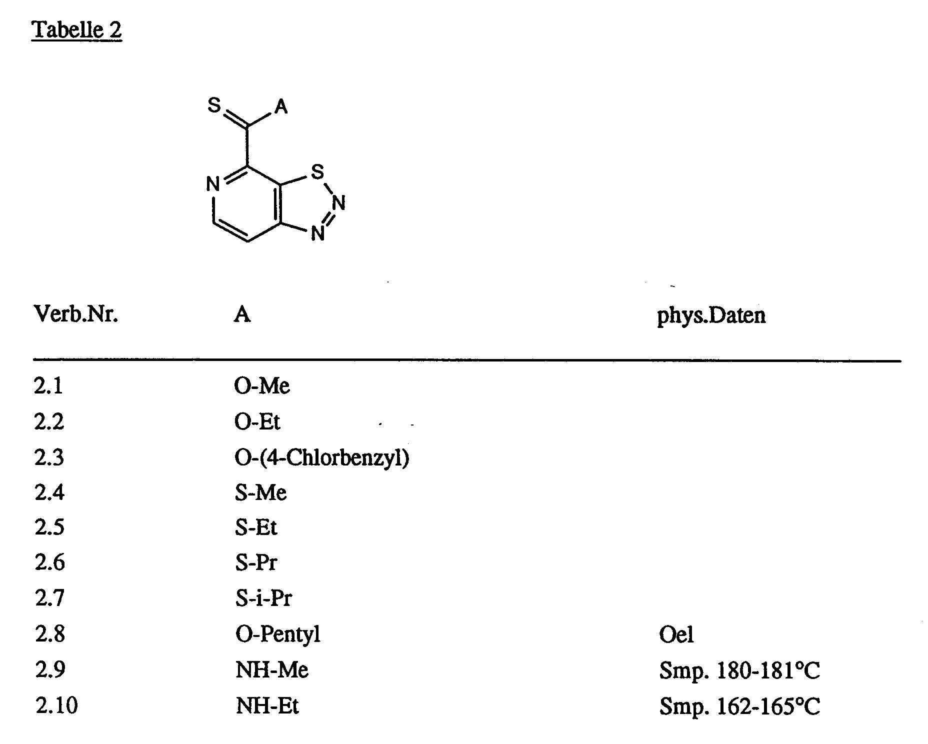

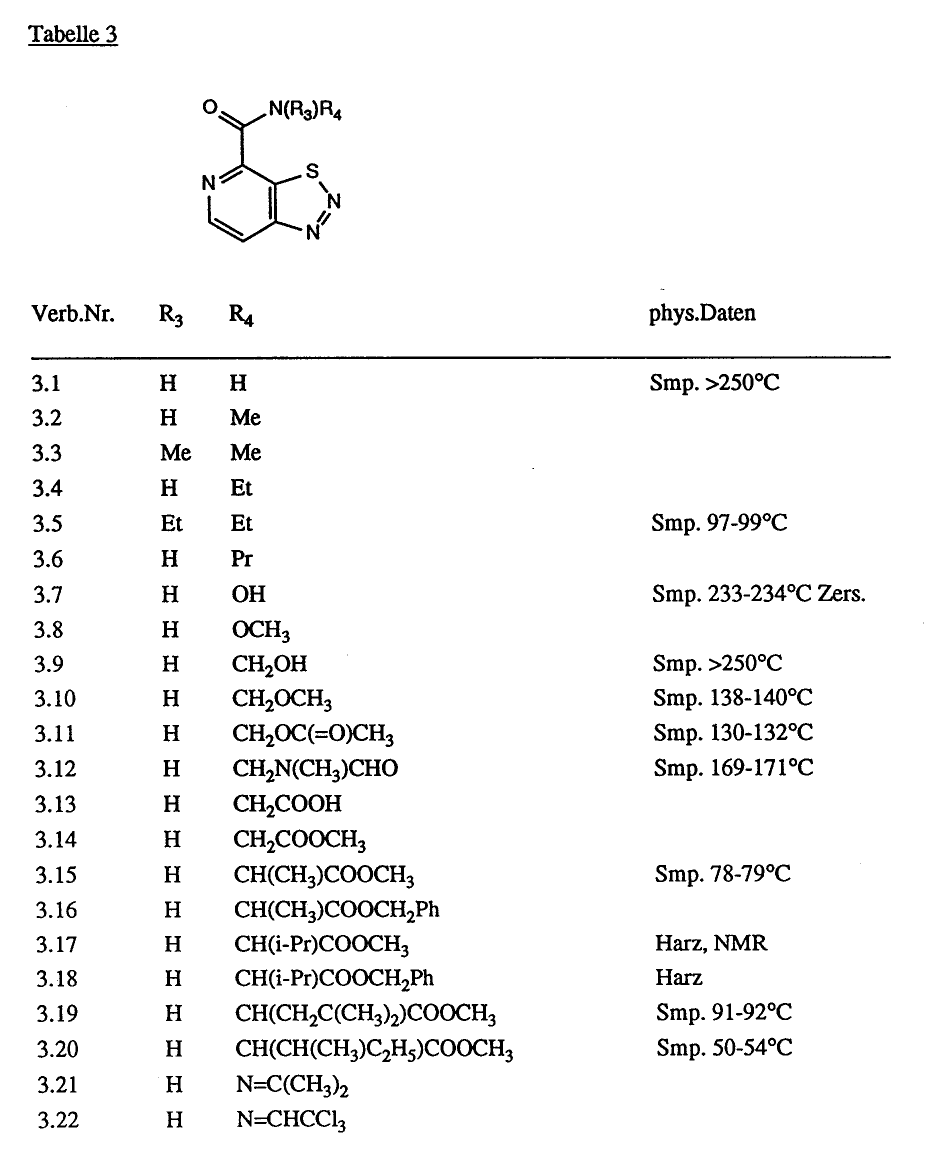

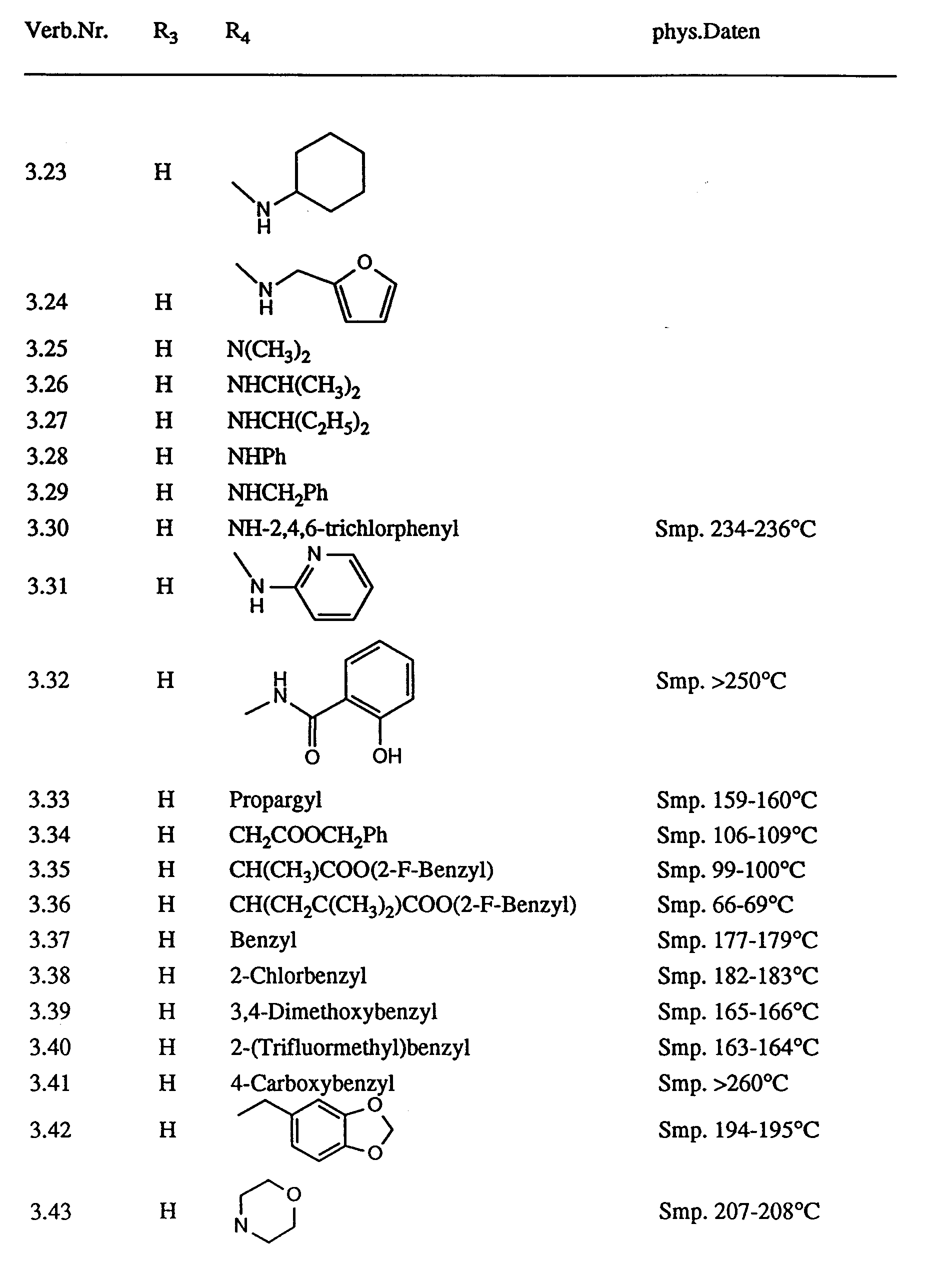

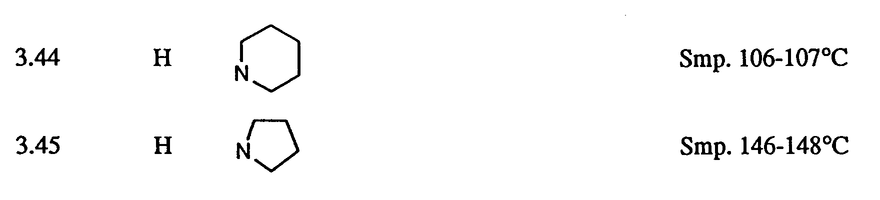

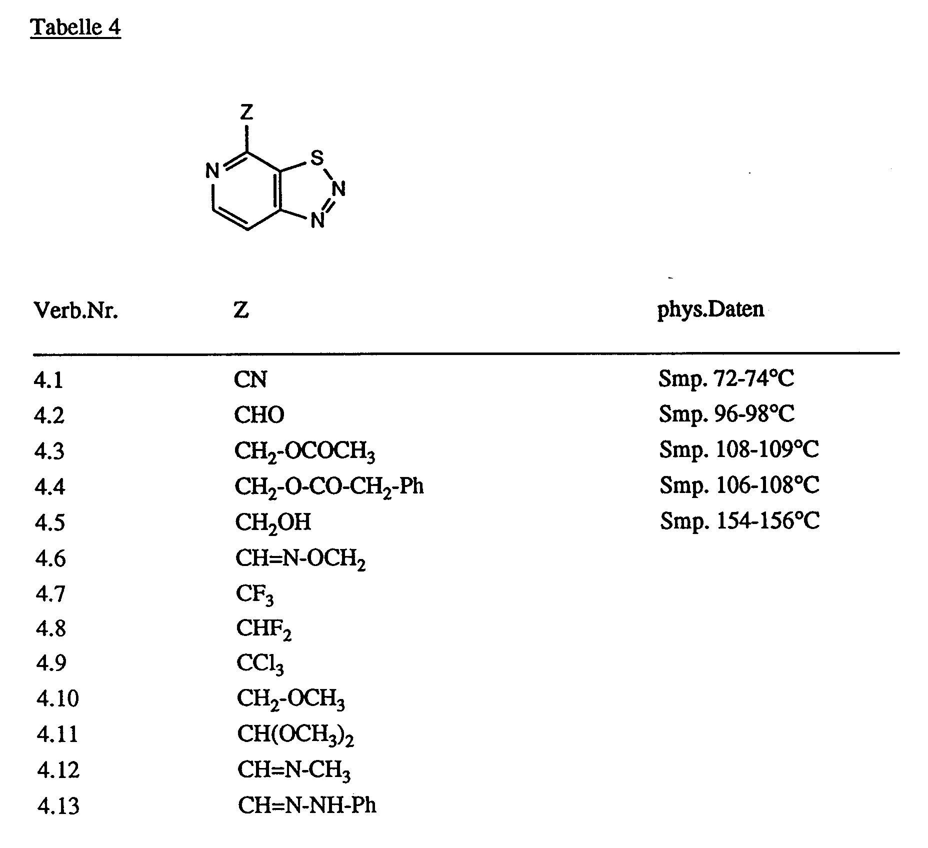

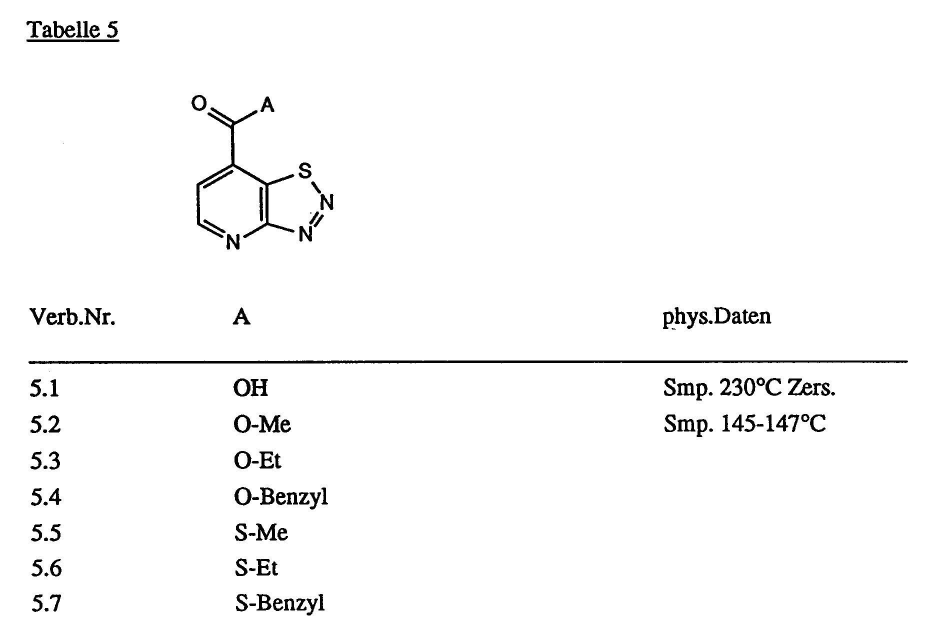

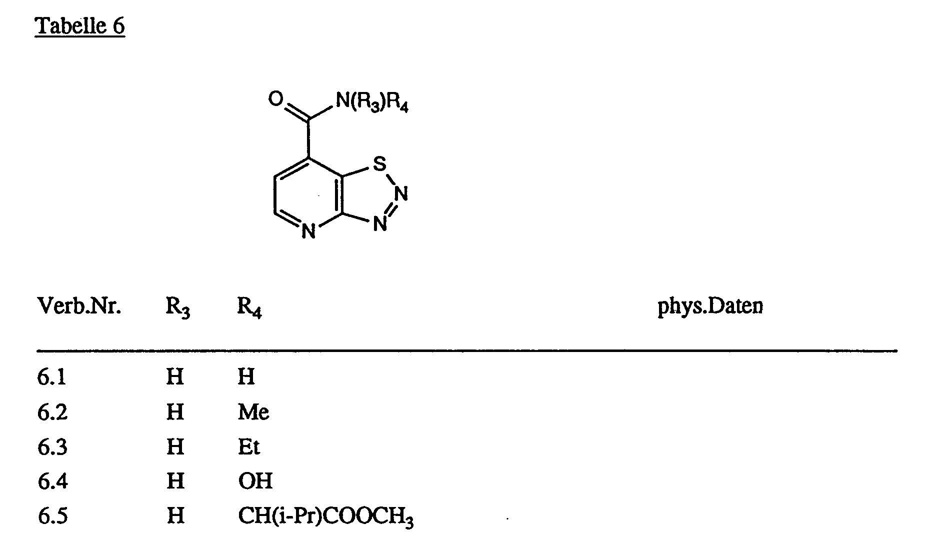

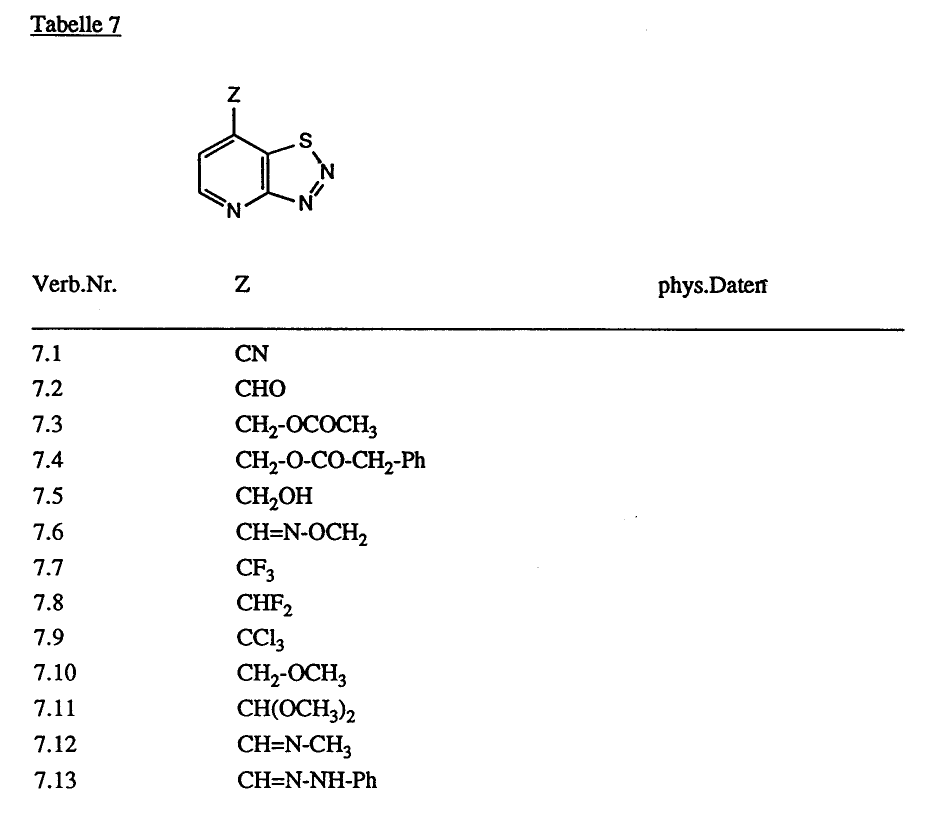

### 2. Formulierungsbeispiele für Wirkstoffe aus den Tabellen (% = Gewichtsprozent)

### 2.1 Spritzpulver

| | a) | b) | c) |
|---|---|---|---|
| Wirkstoff aus den Tabellen | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyethylenglykolether (7-8 Mol Ethylenoxid) | - | 2 % | - |
| Hochdisperse Kieselsäure | 5 % | 10 % | 10 % |
| Kaolin | 62 % | 27 % | - |

Der Wirkstoff wird mit den Zusatzstoffen vermischt und in einer geeigneten Mühle homogen vermahlen. Man erhält Spritzpulver, die sich mit Wasser zu Suspensionen jeder gewünschten Konzentration verdünnen lassen.

### 2.2 Emulsions-Konzentrat

| | |
|---|---|
| Wirkstoff aus den Tabellen | 10 % |
| Octylphenolpolyethylenglykolether (4-5 Mol Ethylenoxid) | 3 % |
| Ca-Dodecylbenzolsulfonat | 3 % |
| Ricinusölpolyglykolether (35 Mol Ethylenoxid) | 4 % |
| Cyclohexanon | 30 % |
| Xylolgemisch | 50 % |

Aus diesem Konzentrat können durch Verdünnen mit Wasser Emulsionen jeder gewünschten Verdünnung hergestellt werden.

### 2.3 Stäubemittel

| | a) | b) |
|---|---|---|
| Wirkstoff aus den Tabellen | 5 % | 8 % |
| Talkum | 95 % | - |
| Kaolin | - | 92 % |

Man erhält anwendungsfertige Stäubemittel, indem der Wirkstoff mit den Trägerstoffen vermischt und auf einer geeigneten Mühle vermahlen wird.

### 2.4 Extruder Granulat

| | |
|---|---|
| Wirkstoff aus den Tabellen | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

### 2.5 Umhüllungs-Granulat

| | |
|---|---|
| Wirkstoff aus den Tabellen | 3 % |
| Polyethylenglykol (MG 200) | 3 % |
| Kaolin | 94 % |
| (MG = Molekulargewicht) | |

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

### 2.6 Suspensions-Konzentrat

| | |
|---|---|
| Wirkstoff aus den Tabellen | 40 % |
| Ethylenglykol | 10 % |
| Nonylphenolpolyethylenglykolether (15 Mol Ethylenoxid) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält ein Suspensions-Konzentrat, aus welchem durch Verdünnen mit Wasser Suspensionen jeder gewünschten Verdünnung hergestellt werden können.

### 3. Biologische Beispiele

### Beispiel 3.1: Wirkung gegen Colletotrichum lagenarium auf Cucumis sativus L.

a) Gurkenpflanzen werden nach 2-wöchiger Anzucht mit einer aus Spritzpulver des Wirkstoffes hergestellten Spritzbrühe besprüht (Konzentration: 200 ppm). Nach 72 Stunden werden die Pflanzen mit einer Sporensuspension (1.0 · 10⁵ Sporen/ml) des Pilzes infiziert und für 30 Stunden bei hoher Luftfeuchtigkeit und einer Temperatur von 23°C inkubiert. Die Inkubation wird dann bei normaler Luftfeuchtigkeit und bei 22°C bis 23°C weitergeführt.
   Die Beurteilung der Schutzwirkung erfolgt aufgrund des Pilzbefalls 7-8 Tage nach der Infektion.
b) Gurkenpflanzen werden nach 2-wöchiger Anzucht mit einer aus Spritzpulver des Wirkstoffes hergestellten Spritzbrühe durch Bodenapplikation behandelt (Konzentration: 20 ppm bezogen auf das Bodenvolumen). Nach 72 Stunden werden die Pflanzen mit einer Sporensuspension (1.5 · 10⁵ Sporen/ml) des Pilzes infiziert und für 30 Stunden bei hoher Luftfeuchtigkeit und einer Temperatur von 23°C inkubiert Die Inkubation wird dann bei normaler Luftfeuchtigkeit und bei 22°C weitergeführt.
   Die Beurteilung der Schutzwirkung erfolgt aufgrund des Pilzbefalls 7-8 Tage nach der Infektion.
   Verbindungen aus den Tabellen zeigen in den Tests (a) und (b) gute Wirkung und reduzieren den Pilzbefall auf 0 bis 20 %. Unbehandelte jedoch infizierte Kontrollpflanzen weisen dagegen einen Colletotrichum-Befall von 100 % auf.

### Beispiel 3.2: Wirkung gegen Phytophthora infestans auf Tomatenpflanzen

a) Tomatenpflanzen werden nach 3-wöchiger Anzucht mit einer aus Spritzpulver des Wirkstoffes hergestellten Spritzbrühe (0,02 % Aktivsubstanz) besprüht. Nach 72 Stunden werden die behandelten Pflanzen mit einer Sporangiensuspension des Pilzes infiziert. Die Beurteilung des Pilzbefalls erfolgt nach einer Inkubation der infizierten Pflanzen während 5 Tagen bei 9O-100 % relativer Luftfeuchtigkeit und 20°C.
   Verbindungen aus den Tabellen zeigen in den Tests gute Wirkung und reduzieren den Pilzbefall auf 0 bis 20 %. Unbehandelte jedoch infizierte Kontrollpflanzen weisen dagegen einen 100 %igen Phytophthora-Befall auf.

### Beispiel 3.3: Wirkung gegen Pyricularia oryzae auf Reispflanzen

Zu 2-wöchigen Reispflanzen wird eine aus Spritzpulver des Wirkstoffes hergestellte Spritzbrühe gegossen (0,006 % Aktivsubstanz bezogen auf das Erdvolumen). Darauf werden die Töpfe mit Wasser soweit gefüllt, dass die untersten Stengelteile der Reispflanzen im Wasser stehen. Nach 96 Stunden werden die behandelten Reispflanzen mit einer Konidiensuspension des Pilzes infiziert. Nach einer Inkubation der infizierten Pflanzen während 5 Tagen bei 95 - 100 % relativer Luftfeuchtigkeit und ca. 24°C wird der Pilzbefall beurteilt.
Reispflanzen, die mit einer Spritzbrühe behandelt werden, die als Aktivsubstanz eine Verbindung aus den Tabellen enthält, zeigen im Vergleich zu unbehandelten Kontrollpflanzen (100 % Befall) nur etwa 50% Pilzbefall.

### Beispiel 3.4: Wirkung gegen Peronospora tabacina an Tabakpflanzen

### a) Blattapplikation

Tabakpflanzen (8 Wochen alt) werden mit einer formulierten Lösung des Wirkstoffes besprüht (Konzentration: 0,02 % Aktivsubstanz). Vier Tage nach der Behandlung werden die Pflanzen mit einer Sporangiensuspension von Peronospora tabacina (100 Sporangien/ml) inokuliert, 20 Stunden im Dunkeln bei 25°C und hoher Luftfeuchtigkeit aufbewahrt und dann bei normaler Tag/Nacht Wechselfolge weiterinkubiert.
Die Beurteilung der Symptome in den Tests erfolgt aufgrund der mit Pilz befallenen Blattoberfläche.
Die Kontrollpflanzen zeigen einen Befall von 90 bis 100 %. Pflanzen, welche mit Verbindungen aus den Tabellen behandelt wurden, zeigen einen Befall von 0-30 %.

### Beispiel 3.5: Wirkung gegen Erysiphe graminis auf Weizen

Protektive Wirkung: 18 Tage alte Weizenpflanzen werden mit einer formulierten Lösung des Wirkstoffes (0,02 % Aktivsubstanz) besprüht. Unmittelbar nach der Behandlung werden die Pflanzen unter Zylindern inkubiert. Nach 24 Stunden werden die Pflanzen abgedeckt. Nach weiteren 3 Tagen werden die behandelten Pflanzen oberhalb vom Primärblatt abgeschnitten. Die Primärblätter werden horizontal orientiert und in einer Bestäubungsglocke mit Eryiphe graminis-Sporen inokuliert (Sporendichte: 0,2 mg pro m²). Der Test wird in einer Klimakammer bei 12h Licht (18 KLux), 20°C und 12h Dunkelheit, 18°C durchgeführt.
Die Beurteilung des Befalls erfolgt 9 und 13 Tage nach Inokulation.
Verbindungen aus den Tabellen zeigen in den Tests gute Wirkung und reduzieren den Pilzbefall auf 0 bis 20 %. Unbehandelte jedoch infizierte Kontrollpflanzen weisen dagegen einen 100 %igen Erysiphe-Befall auf.

### Beispiel 3.6: Wirkung gegen Puccinia graminis auf Weizen

6 Tage alte Weizenpflanzen werden mit einer formulierten Lösung des Wirkstoffes (0.02% Wirkstoff) besprüht. Nach 24 Stunden werden die Pflanzen mit einer Suspension aus Uredosporen (100'000 pro ml) infiziert. 10 Tage später wird der Befall bestimmt. Verbindungen aus den Tabellen zeigen in den Tests gute Wirkung und reduzieren den Pilzbefall auf 0 bis 20 %. Unbehandelte jedoch infizierte Kontrollpflanzen weisen dagegen einen 100 %igen Puccinia-Befall auf.

## Patentansprüche

1. Eine Verbindung der Formel I 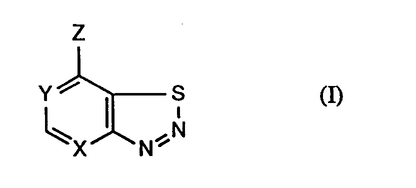 worin
a) X CH und
Y N; oder
b) X N und
Y CH; bedeuten und worin
Z eine C₁-Gruppe darstellt, an die 1-3 Halogenatome oder 1-3 gegebenenfalls substituierte Heteroatome O,S und/oder N gebunden sind;
in freier Form oder in Salzform.

2. Eine Verbindung der Formel I gemäss Anspruch 1
worin bedeuten:
Z CN, CO-A, CS-A, CH=U, CH₂-V, CH(-V)₂ oder C(-V)₃;
und worin ferner die übrigen Substituenten folgende Bedeutungen haben:
A OR₂, SR₂, N(R₃)R₄, N(R₅)-OR₆, O-N(=C)ₙ(R₇)R₈ oder N(R₉)-N(=C)ₙ(R₇)R₈;
U O, NR₁₀, N-OR₆ oder N-N(=C)ₙ(R₇)R₈;
V Halogen, Hydroxy, C₁-C₄-Alkoxy, C₁-C₄-Acyloxy, Benzoyloxy, Benzyloxy oder zwei V zusammen mit dem Kohlenstoffatom, an das sie gebunden sind, eine cyclische Acetalgruppe;
n 0 oder 1;
R₂ bis R₁₀ Wasserstoff, ein gegebenenfalls substituierter, bis zu 8 Kohlenstoffatome enthaltender offenkettiger, gesättigter oder ungesättigter Kohlenwasserstoffrest, ein gegebenenfalls substituierter, bis zu 10 Kohlenstoffatome enthaltender cyclischer, gesättigter oder ungesättigter Kohlenwasserstoffrest, gegebenenfalls substituiertes Benzyl oder Phenethyl, eine gegebenenfalls substituierte, bis zu 8 Kohlenstoffatome enthaltende Acylgruppe, eine gegebenenfalls substituierte Benzoylgruppe oder ein gegebenenfalls substituierter Heterocyclylrest; oder
R₃ und R₄ zusammen mit dem Stickstoffatom, an das sie gebunden sind, einen 5- oder 6-gliedrigen, gegebenenfalls substituierten Heterocyclus; oder
R₇ und R₈ zusammen mit dem Atom, an welches sie gebunden sind, einen 5- oder 6-gliedrigen carbocyclischen oder heterocyclischen Ring, wobei die genannten Ringe unsubstituiert oder substituiert sind;
in freier Form oder in Salzform.

3. Eine Verbindung der Formel I gemäss Anspruch 2, worin bedeuten:
R₂ und R₃ Wasserstoff, C₁-C₈-Alkyl, das unsubstituiert oder mit 1-5 Halogenatomen, C₃-C₆-Cycloalkyl, C₁-C₄-Alkoxy, Phenoxy, Hydroxy, Nitro, Cyano, C₁-C₄-Alkanoyl, Benzoyl, Carboxyl, C₁-C₄-Alkoxycarbonyl, Benzyloxycarbonyl, C₁-C₄-Acyloxy, Benzoyloxy, C₁-C₄-Dialkylamino oder Heterocyclyl substituiert ist, C₃-C₆-Alkenyl, das unsubstituiert oder mit 1-5 Halogenatomen substituiert ist, C₃-C₆-Alkinyl, C₃-C₆-Cycloalkyl, Phenyl, Benzyl oder Phenethyl, deren Phenylringe unsubstitiert oder ein bis dreifach durch Halogen, Hydroxy, C₁-C₄-Alkyl, Halogen-C₁-C₂-alkyl, C₁-C₂-Alkoxy, Halogen-C₁-C₂-alkoxy oder Nitro substituiert sind, Naphthyl, C₁-C₄-Alkanoyl, Benzoyl, oder Heterocyclyl, das unsubstituiert oder ein- bis dreifach gleich oder verschieden durch Halogen, C₁-C₂-Alkyl, Halogenmethyl oder Nitro substituiert ist;
R₄ Wasserstoff, C₁-C₆-Alkyl, C₃-C₆-Alkenyl, C₃-C₆-Alkinyl, C₃-C₆-Cycloalkyl, Phenyl oder Benzyl, deren Phenylringe unsubstitiert oder ein bis zweifach durch Halogen, Hydroxy, C₁-C₂-Alkyl, C₁-C₂-Alkoxy, Halogenmethyl oder Nitro substituiert sind; oder
R₃ und R₄ zusammen mit dem Stickstoffatom, an das sie gebunden sind, einen 5- oder 6-gliedrigen Heterocyclus, der unsubstituiert oder ein- bis dreifach durch C₁-C₂-Alkyl substituiert ist;
R₅, R₆, R₇, R₈, R₉ und R₁₀ unabhängig voneinander Wasserstoff, C₁-C₆-Alkyl, C₃-C₆-Alkenyl, C₃-C₆-Alkinyl, C₃-C₆-Cycloalkyl, Phenyl oder Benzyl, deren Phenylringe unsubstitiert oder ein bis zweifach durch Halogen, Hydroxy, C₁-C₂-Alkyl, C₁-C₂-Alkoxy, Halogen-C₁-C₂-alkyl oder Nitro substituiert sind;
oder
R₇ und R₈ zusammen mit dem Atom, an welches sie gebunden sind, einen 5- bis 7-gliedrigen carbocyclischen oder heterocyclischen Ring, wobei die genannten Ringe unsubstituiert oder ein- bis dreifach gleich oder verschieden durch Halogen, C₁-C₂-Alkyl, Halogen-C₁-C₂-alkyl oder Nitro substituiert sind.

4. Eine Verbindung der Formel I gemäss Anspruch 3, worin bedeuten:
R₂ und R₃ Wasserstoff, C₁-C₅-Alkyl, das unsubstituiert oder mit 1-3 Halogenatomen, C₃-C₆-Cycloalkyl, C₁-C₂-Alkoxy, Phenoxy, Hydroxy, Nitro, Cyano, C₁-C₂-Alkanoyl, Benzoyl, Carboxyl, C₁-C₄-Alkoxycarbonyl, Benzyloxycarbonyl, C₁-C₂-Acyloxy, Benzoyloxy, C₁-C₄-Dialkylamino oder Heterocyclyl substituiert ist, C₃-C₄-Alkenyl, das unsubstituiert oder mit 1-3 Halogenatomen substituiert ist, C₃-C₄-Alkinyl, C₃-C₆-Cycloalkyl, Phenyl, Benzyl oder Phenethyl, deren Phenylringe unsubstitiert oder ein bis zweifach durch Halogen, Hydroxy, C₁-C₄-Alkyl, Halogen-C₁-C₂-alkyl, C₁-C₂-Alkoxy, Halogen-C₁-C₂-alkoxy, oder Nitro substituiert sind, Naphthyl oder Heterocyclyl, das unsubstituiert oder ein- bis zweifach gleich oder verschieden durch Halogen, C₁-C₂-Alkyl, Halogenmethyl oder Nitro substituiert ist;
R₄ Wasserstoff, C₁-C₄-Alkyl, C₃-C₄-Alkenyl, C₃-C₄-Alkinyl, C₃-C₆-Cycloalkyl, Phenyl oder Benzyl;
R₃ und R₄ zusammen mit dem Stickstoffatom, an das sie gebunden sind, Pyrrolidin, Piperidin, Morpholin oder Dimethylmorpholin;
R₅, R₆, R₇, R₈, R₉und R₁₀ unabhängig voneinander Wasserstoff, C₁-C₄-Alkyl, C₃-C₄-Alkenyl, C₃-C₄-Alkinyl, C₃-C₆-Cycloalkyl, Phenyl oder Benzyl, deren Phenylringe unsubstitiert oder ein bis zweifach durch Halogen, Hydroxy, C₁-C₂-Alkyl, C₁-C₂-Alkoxy, Halogenmethyl oder Nitro substituiert sind; oder
R₇ und R₈ zusammen mit dem Atom, an welches sie gebunden sind, einen 5- bis 7-gliedrigen carbocyclischen oder heterocyclischen Ring, wobei die genannten Ringe unsubstituiert oder ein- bis zweifach gleich oder verschieden durch Halogen, Methyl, Halogenmethyl oder Nitro substituiert sind.

5. Eine Verbindung der Formel I gemäss Anspruch 2, worin bedeuten:
Z CO-A;
A OR₂, SR₂, N(R₃)R₄, N(R₅)-OR₆, O-N(=C)ₙ(R₇)R₈ oder N(R₉)-N(=C)ₙ(R₇)R₈.

6. Eine Verbindung der Formel I gemäss Anspruch 5, worin bedeuten:
Z CO-A;
A OR₂ oder SR₂.

7. Eine Verbindung der Formel I gemäss Anspruch 6, worin bedeuten:
R₂ Wasserstoff, C₁-C₅-Alkyl, das unsubstituiert oder mit 1-3 Halogenatomen, C₃-C₆-Cycloalkyl, C₁-C₂-Alkoxy, Phenoxy, Hydroxy, Nitro, Cyano, C₁-C₂-Alkanoyl, Benzoyl, Carboxyl, C₁-C₄-Alkoxycarbonyl, Benzyloxycarbonyl, C₁-C₂-Acyloxy, Benzoyloxy, C₁-C₄-Dialkylamino oder Heterocyclyl substituiert ist, C₃-C₄-Alkenyl, das unsubstituiert oder mit 1-3 Halogenatomen substituiert ist, C₃-C₄-Alkinyl, C₃-C₆-Cycloalkyl, Phenyl, Benzyl oder Phenethyl, deren Phenylringe unsubstitiert oder ein bis zweifach durch Halogen, Hydroxy, C₁-C₄-Alkyl, Halogen-C₁-C₂-alkyl, C₁-C₂-Alkoxy, Halogen-C₁-C₂-alkoxy, oder Nitro substituiert sind, Naphthyl oder Heterocyclyl, das unsubstituiert oder ein- bis zweifach gleich oder verschieden durch Halogen, C₁-C₂-Alkyl, Halogenmethyl oder Nitro substituiert ist;

8. Eine Verbindung der Formel I gemäss Anspruch 7, worin bedeuten:
R₂ Wasserstoff, C₁-C₅-Alkyl,Propenyl, Phenyl oder Benzyl, deren Phenylringe unsubstitiert oder ein bis zweifach durch Halogen substituiert sind.

9. Eine Verbindung der Formel I gemäss Anspruch 2, worin
Z CO-OR₂ bedeutet.

10. Eine Verbindung der Formel I gemäss Anspruch 1, worin bedeuten:
Z CN oder CO-N(R₃)R₄ ;
R₃ Wasserstoff, C₁-C₅-Alkyl, das unsubstituiert oder mit 1-3 Halogenatomen, C₃-C₆-Cycloalkyl, C₁-C₂-Alkoxy, Phenoxy, Hydroxy, Nitro, Cyano, C₁-C₂-Alkanoyl, Benzoyl, Carboxyl, C₁-C₄-Alkoxycarbonyl, Benzyloxycarbonyl, C₁-C₂-Acyloxy, Benzoyloxy, C₁-C₄-Dialkylamino oder Heterocyclyl substituiert ist, C₃-C₄-Alkenyl, das unsubstituiert oder mit 1-3 Halogenatomen substituiert ist, C₃-C₄-Alkinyl, C₃-C₆-Cycloalkyl, Phenyl, Benzyl oder Phenethyl, deren Phenylringe unsubstitiert oder ein bis zweifach durch Halogen, Hydroxy, C₁-C₄-Alkyl, Halogen-C₁-C₂-alkyl, C₁-C₂-Alkoxy, Halogen-C₁-C₂-alkoxy, oder Nitro substituiert sind, Naphthyl oder Heterocyclyl, das unsubstituiert oder ein- bis zweifach gleich oder verschieden durch Halogen, C₁-C₂-Alkyl, Halogenmethyl oder Nitro substituiert ist;
R₄ Wasserstoff, C₁-C₄-Alkyl, C₃-C₄-Alkenyl, C₃-C₄-Alkinyl, C₃-C₆-Cycloalkyl, Phenyl oder Benzyl;
R₃ und R₄ zusammen mit dem Stickstoffatom, an das sie gebunden sind, Pyrrolidin, Piperidin, Morpholin oder Dimethylmorpholin.

11. Eine Verbindung der Formel I gemäss Anspruch 10, worin bedeuten:
Z CO-N(R₃)R₄;
R₃ Wasserstoff, C₁-C₅-Alkyl, das unsubstituiert oder mit C₁-C₄-Alkoxycarbonyl oder Benzyloxycarbonyl substituiert ist, C₃-C₄-Alkenyl, C₃-C₄-Alkinyl, C₃-C₆-Cycloalkyl, Phenyl oder Benzyl, deren Phenylringe unsubstitiert oder ein bis zweifach durch Halogen, Hydroxy, Methyl, Methoxy, Halogenmethyl, Halogenmethoxy oder Nitro substituiert sind;
R₄ Wasserstoff oder Methyl; oder
R₃ und R₄ zusammen mit dem Stickstoffatom, an das sie gebunden sind, Pyrrolidin, Piperidin, Morpholin oder Dimethylmorpholin.

12. Eine Verbindung der Formel I gemäss Anspruch 1, worin bedeuten:
Z CS-A;
A OR₂, SR₂ oder N(R₃)R₄;
R₂ und R₃ Wasserstoff, C₁-C₅-Alkyl, das unsubstituiert oder mit 1-3 Halogenatomen, C₃-C₆-Cycloalkyl, C₁-C₂-Alkoxy, Phenoxy, Hydroxy, Nitro, Cyano, C₁-C₂-Alkanoyl, Benzoyl, Carboxyl, C₁-C₄-Alkoxycarbonyl, Benzyloxycarbonyl, C₁-C₂-Acyloxy, Benzoyloxy, C₁-C₄-Dialkylamino oder Heterocyclyl substituiert ist, C₃-C₄-Alkenyl, das unsubstituiert oder mit 1-3 Halogenatomen substituiert ist, C₃-C₄-Alkinyl, C₃-C₆-Cycloalkyl, Phenyl, Benzyl oder Phenethyl, deren Phenylringe unsubstitiert oder ein bis zweifach durch Halogen, Hydroxy, C₁-C₄-Alkyl, Halogen-C₁-C₂-alkyl, C₁-C₂-Alkoxy, Halogen-C₁-C₂-alkoxy, oder Nitro substituiert sind, Naphthyl oder Heterocyclyl, das unsubstituiert oder ein- bis zweifach gleich oder verschieden durch Halogen, C₁-C₂-Alkyl, Halogenmethyl oder Nitro substituiert ist;
R₄ Wasserstoff, C₁-C₄-Alkyl, C₃-C₄-Alkenyl, C₃-C₄-Alkinyl, C₃-C₆-Cycloalkyl, Phenyl oder Benzyl;
R₃ und R₄ zusammen mit dem Stickstoffatom, an das sie gebunden sind, Pyrrolidin, Piperidin, Morpholin oder Dimethylmorpholin.

13. Eine Verbindung der Formel I gemäss Anspruch 2, worin bedeuten:
Z CN, CH=U oder CH₂-V;
U O, NR₁₀, N-OR₆ oder N-N(=C)ₙ(R₇)R₈;
V Halogen, Hydroxy, C₁-C₄-Alkoxy, C₁-C₄-Acyloxy, Benzoyloxy, Benzyloxy; und
worin
R₆,R₇,R₈ und R₁₀ die in Anspruch 2 angegebenen Bedeutungen haben;
n 0 oder 1.

14. Eine Verbindung der Formel I gemäss Anspruch 1, worin bedeuten:
Z CH(V)₂;
V C₁-C₄-Alkoxy oder ein 5-oder 6-gliedriges cyclisches Acetal.

15. Eine Verbindung der Formel I gemäss Anspruch 1, worin bedeuten:
Z CN, COOH, CHO, CH₂OH, CH₂Cl, CCl₃ CHF₂ oder CF₃.

16. Eine Verbindung gemäss den Ansprüchen 1 bis 15 der Formel Ia

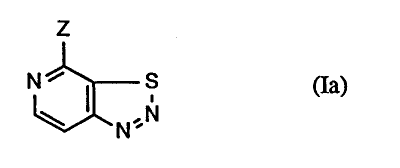

17. Eine Verbindung gemäss den Ansprüchen 1 bis 15 der Formel Ib

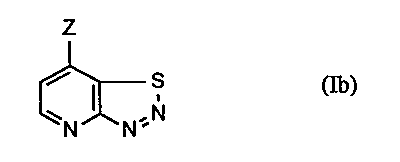

18. Verfahren zur Herstellung einer Verbindung der Formel Ia gemäss Anspruch 16, dadurch gekennzeichnet, dass man eine Verbindung der Formel II 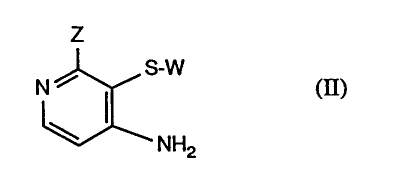 worin Z die für Formel I angegebenen Bedeutungen hat und
W einen gegebenenfalls substituierten C₁-C₁₂-Kohlenwasserstoff oder eine gegebenenfalls substituierte Carbamoylgruppe bedeutet, mit einem anorganischen oder organischen Nitrit in einem geeigneten Lösungsmittel umsetzt.

19. Verfahren zur Herstellung einer Verbindung der Formel Ib gemäss Anspruch 17, dadurch gekennzeichnet, dass man eine Verbindung der Formel XXIII, 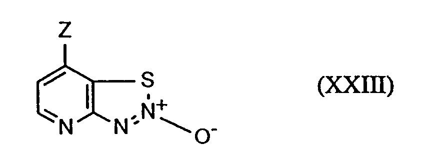 worin Z die für Formel I angegebenen Bedeutungen hat,
mit einem Reduktionsmittel umsetzt.

20. Mittel zum Schutz von Pflanzen gegen den Befall durch Mikroorganismen, dadurch gekennzeichnet, dass es als Wirkstoff mindestens eine Verbindung gemäss Anspruch 1, in freier Form oder in agrochemisch verwendbarer Salzform, zusammen mit einem Trägermaterial enthält.

21. Verfahren zum Schutz von Pflanzen gegen den Befall durch Mikroorganismen, dadurch gekennzeichnet, dass man als Wirkstoff eine Verbindung der Formel I gemäss Anspruch 1 auf die Pflanzen, auf Teile der Pflanzen und/oder auf den Standort der Pflanzen appliziert.

22. Verfahren zur Immunisierung von Pflanzen gegen den Befall durch Mikroorganismen, dadurch gekennzeichnet, dass man als Wirkstoff eine Verbindung der Formel I gemäss Anspruch 1 auf die Pflanzen, auf Teile der Pflanzen und/oder auf den Standort der Pflanzen appliziert.

23. Eine Verbindung der Formel XXIII, 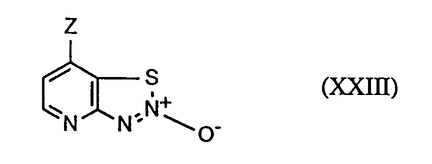 worin Z die für Formel I angegebenen Bedeutungen hat.
